(19) Europäisches Patentamt
European Patent Office
Office européen des brevets

(11) EP 4 306 126 A1

(12) EUROPEAN PATENT APPLICATION
published in accordance with Art. 153(4) EPC

(43) Date of publication:
**17.01.2024 Bulletin 2024/03**

(21) Application number: **21930477.1**

(22) Date of filing: **11.11.2021**

(51) International Patent Classification (IPC):
***A61K 39/215*** *(2006.01)* ***A61K 9/127*** *(2006.01)*
***A61K 9/51*** *(2006.01)* ***A61K 47/28*** *(2006.01)*
***A61K 9/19*** *(2006.01)* ***A61P 31/14*** *(2006.01)*
***A61K 48/00*** *(2006.01)* ***A61K 39/00*** *(2006.01)*

(52) Cooperative Patent Classification (CPC):
**A61K 9/127; A61K 9/19; A61K 9/51; A61K 39/00; A61K 39/215; A61K 47/28; A61K 48/00; A61P 31/14**

(86) International application number:
**PCT/KR2021/016460**

(87) International publication number:
**WO 2022/191377 (15.09.2022 Gazette 2022/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.03.2021 KR 20210029928**
**13.08.2021 KR 20210107267**

(71) Applicant: **Eyegene Inc.**
**Seoul 07528 (KR)**

(72) Inventors:
- **CHO, Yang Je**
**Seoul 04423 (KR)**
- **KIM, Seok Hyun**
**Goyang-si Gyeonggi-do 10416 (KR)**
- **KIM, Kwangsung**
**Goyang-si Gyeonggi-do 10371 (KR)**

(74) Representative: **Mewburn Ellis LLP**
**Aurora Building**
**Counterslip**
**Bristol BS1 6BX (GB)**

# (54) VACCINE COMPOSITION FOR PREVENTING SARS-COV-2

(57) The present invention relates to a vaccine composition for preventing SARS-CoV-2, comprising mRNA encoding an S mutant antigen of SARS-CoV-2 virus, wherein a vaccine for preventing SARS-CoV-2 according to the present invention exhibits excellent stability and high immunogenicity *in vivo,* and the vaccine is thus easy to store and use, and excellent preventive effect thereof against COVID-19 can be expected.

EP 4 306 126 A1

FIG. 9

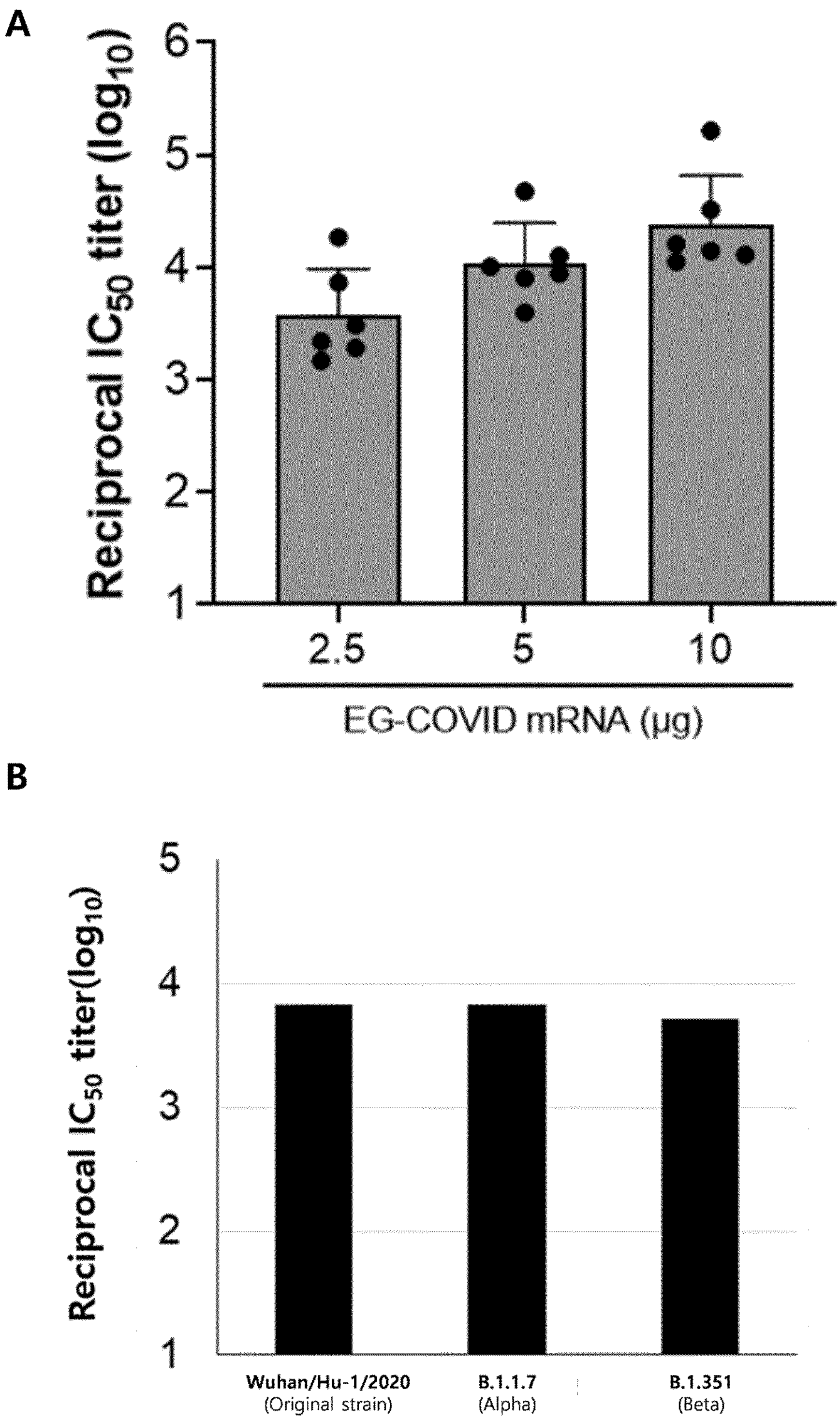
A
Reciprocal $IC_{50}$ titer ($log_{10}$)
6
5
4
3
2
1
2.5
5
10
EG-COVID mRNA (μg)
B
Reciprocal $IC_{50}$ titer($log_{10}$)
5
4
3
2
1
Wuhan/Hu-1/2020
(Original strain)
B.1.1.7
(Alpha)
B.1.351
(Beta)

## Description

### Technical Field

The present invention relates to a vaccine composition for preventing SARS-CoV-2, and more particularly to a vaccine composition for preventing SARS-CoV-2 comprising mRNA encoding the variant S antigen of SARS-CoV-2 virus.

### Background Art

Coronavirus is a type of RNA virus, genetic information of which is composed of ribonucleic acid (RNA). Coronavirus causes respiratory and gastrointestinal infections in humans and animals. Coronavirus is easily transmitted mainly by mucosal transmission, droplet transmission, etc., and generally causes mild respiratory infections in humans, but unusually causes fatal infections.

SARS-CoV-2 (severe acute respiratory syndrome coronavirus 2), which is currently causing the pandemic, is a positive-sense single-stranded RNA coronavirus, is contagious to humans, and is the cause of coronavirus disease 2019 (COVID-19). SARS-CoV-2 virus binds to ACE2 (angiotensin-converting enzyme 2) present on the surface of airway epithelial cells, alveolar epithelial cells, vascular endothelial cells, and macrophages in the lungs using the spike protein on the surface thereof, and thus invades into the host cells. Based on results of genome sequencing of SARS-CoV-2, a receptor-binding domain (RBD) with a tertiary structure very similar to that of SARS-CoV was identified in the spike protein, and it was speculated that the RBD of SARS-CoV-2 has higher binding affinity for ACE2 than the RBD of SARS-CoV and thus the contagiousness of SARS-CoV-2 is stronger than that of SARS-CoV (Matthew Z T et al., Nature Reviews Immunology, 20:363-374, 2020).

Spike protein consists of two proteins, S1 and S2, among which the S1 protein is composed of an amino-terminal domain and RBD. When RBD binds to ACE2, SARS-CoV-2 virions enter the cell's endosome through endocytosis, after which the fusion peptide is exposed and inserted into the membrane of the host cells. The S2 protein is composed of a fusion peptide region (FP region) and heptad repeat regions (HR1, HR2), and HR1 and HR2 are fused to the viral membrane in a contacting manner, so that SARS-CoV-2 virions are released outside the host cells. S1 and S2 have different cleavage sites and are cleaved by respective proteases, resulting in SARS-CoV-2 infection. SARS-CoV-2 therapeutics and vaccines are being developed using strategies that inhibit S1 and S2 cleavage or disrupt the binding between proteins such as ACE2 or TMPRSS2 (transmembrane serine protease 2) and the virus (Matthew Z T et al., Nature Reviews Immunology, 20:363-374, 2020) .

Since the spike protein of SARS-CoV-2 has an unstable protein structure, misfolding or triggering is prevented through proline substitution for 986(K) and 987(V), so that prefusion stabilized viral glycoprotein acts as a better immunogen, as confirmed by previous studies on MERS-CoV and SARS-CoV (Jesper Pallesen et al. PNAS, 2017, DOI: https://doi.org/10.1073/PNAS.1707304114).

Meanwhile, SARS-CoV-2 has been mutated from 'type D (D614)' that originated in Wuhan to 'European type' or 'type G (G614)' based on global initiative on sharing avian influenza data (GISAID). The G-type mutation is characterized by a mutation of the $614^{th}$ amino acid of the viral surface spike protein from aspartic acid (GAT; Asp, D) to glycine (GGT; Gly, G) (FIG. 1) (Plante, J. A et al. Nature (2020). DOI: https://doi.org/10.1038/s41586-020-2895-3).

Due to the global pandemic, vaccines to prevent SARS-CoV-2 virus infection have been developed one after another, and vaccination is in the beginning stage, but the development of a vaccine that exhibits high immunogenicity while maintaining a more stable effect is still required.

Meanwhile, gene therapy and genetic vaccination are technologies that have already been proven in the field of medicine and are generally applied, and may be used to treat not only genetic diseases, but also autoimmune diseases, infectious diseases, cancer or tumor-related diseases, and inflammatory diseases.

Genetic vaccines began to be developed since it was reported that, when DNA and RNA encoding a target gene are directly injected into an animal, the target gene is expressed in the living animal, and immunity is possible by this expression (Wolff JA et al. Science, 247:1465-8, 1990).

Genetic vaccination evokes the desired immune response against selected antigens, such as characteristic components of bacterial surfaces, viral particles, tumor antigens, and the like. All in all, vaccination is one of the pivotal achievements of modern medicine. However, effective vaccines are currently only available for a limited number of diseases. Thus, infections that cannot be prevented by vaccination still affect millions of people each year.

In gene therapy or genetic vaccination, DNA and RNA may be used as nucleic acid molecules for gene administration, and it is known that DNA is relatively stable and easy to handle compared to RNA. However, in the case of DNA, a potential risk may arise if the DNA segment administered into the patient's genome is inserted at an undesirable location and the gene is damaged. Additionally, unwanted anti-DNA antibodies may appear, and another problem is that the expression level of the peptide or protein expressed by DNA administration and subsequent transcription/translation is limited. The presence or absence of a specific transcription factor that regulates DNA transcription has a major impact on the expression level of the administered DNA, and in the absence of a specific transcription factor, a sufficient amount of RNA is not produced by DNA transcription, and consequently, the level of peptide or protein produced by translation is also limited.

On the other hand, when RNA is used as a tool for gene administration, RNA does not require transcription and is thus able to directly synthesize proteins in the cytoplasm without the need to enter the nucleus like DNA, so there is no fear of intruding into the cell chromosome and causing unwanted genetic damage. Moreover, RNA has a shorter half-life than DNA and thus does not induce long-term genetic modification (Sayour EJ, et al., J Immunother Cancer 2015; 3:13, 2015). When delivered into a cell, a general RNA vaccine is activated for a short period of time to express a target protein and is destroyed by an enzymatic reaction within a few days, and a specific immune response to the expressed target antigen (protein) remains.

In addition, when using RNA as a tool for gene administration, RNA works only when it passes through the cell membrane without the need to pass through the nuclear membrane, making it possible to express the same level of the target protein as when using DNA even in a smaller amount than DNA. Moreover, RNA itself has immune-reinforcing activity and is thus capable of exhibiting the same immune effect even when administered in a small amount compared to DNA.

By using RNA instead of DNA for genetic vaccination, the risk of unwanted integration into the genome and the production of anti-DNA antibodies may be minimized or avoided. However, RNA is a highly unstable molecular species that may be readily degraded by ubiquitous RNases.

Although many advances have been made in the past years, there remains a need in the art to provide a method in which the effectiveness of administration is not compromised by inefficient translation of mRNA due to premature degradation of antigen or inefficient release of mRNA from cells, as an efficient method for mRNA vaccination that may induce an immune response. Furthermore, there is also a need to reduce the dose of mRNA vaccines in order to reduce potential safety concerns and to make vaccines affordable throughout the third world.

There are still many issues to be solved with regard to nucleic acid delivery to obtain a desired response in a biological system. Nucleic acid-based therapeutics hold tremendous promise, but realizing this promise requires effective delivery of nucleic acids to appropriate sites within cells or organisms.

However, the use of nucleic acids for therapeutic and prophylactic purposes currently faces two problems. First, free RNA is susceptible to degradation by nucleases in plasma. Second, free RNA has limited ability to access intracellular compartments where the translational machinery that translates the same resides. Incorporating lipid nanoparticles formed from cationic lipids and other lipid components such as neutral lipids, cholesterol, PEG, pegylated lipids, and oligonucleotides has been attempted to block degradation of RNA in plasma and promote cellular uptake of nucleic acids.

Accordingly, the present inventors have made great efforts to develop a preventive vaccine against SARS-CoV-2 with superior storage stability and high immunogenicity *in vivo,* and thus developed an mRNA vaccine loaded with a nucleic acid encoding a variant antigen for the spike protein of SARS-CoV-2 in lipid nanoparticles (LNPs) or liposomes having a specific lipid composition and ascertained that the vaccine exhibits superior stability and high immunogenicity *in vivo,* thus culminating in the present invention.

## Summary of the Invention

It is an object of the present invention to provide a vaccine composition for preventing SARS-CoV-2, which is named EG-COVID and exhibits superior stability and high immunogenicity.

In order to accomplish the above object, the present invention provides a vaccine composition for preventing SARS-CoV-2 comprising mRNA encoding the S antigen of SARS-CoV-2 virus.

The vaccine composition according to the present invention may further comprise liposomes or lipid nanoparticles, and the liposomes or lipid nanoparticles may comprise a cationic lipid, a neutral lipid, and cholesterol.

In addition, the present invention provides a method of preventing SARS-CoV-2 infection comprising administering a composition for preventing SARS-CoV-2 comprising mRNA encoding the S antigen of SARS-CoV-2 virus.

In addition, the present invention provides the use of the composition comprising mRNA encoding the S antigen of SARS-CoV-2 virus for the prevention of SARS-CoV-2 infection.

In addition, the present invention provides the use of the composition for preventing SARS-CoV-2 comprising mRNA encoding the S antigen of SARS-CoV-2 virus for the manufacture of a medicament for the prevention of SARS-CoV-2 infection.

## Brief Description of Drawings

FIG. 1 shows D-type (D614) and G-type (G614) mutations of SARS-CoV-2.

FIG. 2 shows results confirming the delivery efficiency of liposomes having different lipid compositions to mice.

FIG. 3 shows the mRNA expression efficiency in mice depending on the mixing ratio of mRNA and liposomes in an mRNA-liposome complex.

FIG. 4 shows results confirming whether a formulation obtained by mixing CV-LP-b1 with CV-SF-614Gm is normally expressed in HEK293T cells, FIG. 4A showing the expression of SARS-CoV-2 spike protein after treating HEK293T cells with CV-SF-614Gm at different concentrations using lipofectamine, and FIG. 4B showing the expression of SARS-CoV-2 spike protein after treating HEK293T cells with an mRNA complex prepared by lyophilization of a mixture of CV-LP-b1 and CV-SF-614Gm.

FIG. 5A shows the RBD-specific IgG antibody titer analyzed by ELISA in the serum of mice administered with an mRNA complex in which CV-LP-b1 and CV-SF-614Gm were mixed, FIG. 5B shows results of analyzing the concentration of IFN-γ secreted in the medium by ELISA after splenocytes isolated from mice administered with an mRNA complex in which CV-LP-b1 and CV-SF-614Gm were mixed were stimulated with S1 peptide, and FIG. 5C shows the neutralization capacity of the obtained serum analyzed using a SARS-CoV-2 surrogate virus neutralization test (sVNT) kit.

FIG. 6A shows the RBD-specific IgG antibody titer analyzed by ELISA in the serum of mice administered once or twice with an mRNA complex in which CV-LP-b1 and CV-SF-614Gm were mixed, FIG. 6B shows results of analyzing the concentration of IFN-γ secreted in the medium by ELISA after splenocytes isolated from mice administered once or twice with an mRNA complex in which CV-LP-b1 and CV-SF-614Gm were mixed were stimulated with S1 peptide, and FIG. 6C shows the neutralization capacity of the obtained serum analyzed using a SARS-CoV-2 surrogate virus neutralization test (sVNT) kit.

FIG. 7A shows the RBD-specific IgG antibody titer analyzed by ELISA in the serum of mice administered with LEG-COVID as a liquid formulation and F-EG-COVID as a lyophilized formulation refrigerated for 0, 4, and 8 weeks, FIG. 7B shows results of analyzing the concentration of IFN-γ secreted in the medium by ELISA after splenocytes of mice immunized above were stimulated with S1 peptide, and FIG. 7C shows the neutralization capacity of the obtained serum analyzed using a SARS-CoV-2 surrogate virus neutralization test (sVNT) kit.

FIGs. 8a and 8b show results confirming the biodistribution pattern of CV-SF-614Gm over time after intramuscular administration of EG-COVID to rats.

FIG. 9 shows results confirming the evaluation of the neutralizing antibody titer of EG-COVID, in which A shows results of measurement of $IC_{50}$ of EG-COVID based on the SARS-CoV-2 (NCCP 43326) virus infection inhibitory efficiency depending on the concentration of CV-SF-614Gm mRNA and B shows results of measurement of $IC_{50}$ of EG-COVID for Alpha and Beta variants.

**Detailed Description and Preferred Embodiments of the Invention**

Unless otherwise defined, all technical and scientific terms used herein have the same meanings as typically understood by those skilled in the art to which the present invention belongs. In general, the nomenclature used herein is well known in the art and is typical.

An aspect of the present invention pertains to a vaccine composition for preventing SARS-CoV-2 comprising mRNA encoding the S antigen of SARS-CoV-2 virus.

In the present invention, the S antigen of SARS-CoV-2 virus is used as a concept including a wild-type S antigen and a variant S antigen including at least one amino acid mutation.

In particular, in the present invention, the S antigen of SARS-CoV-2 virus is preferably a sequence (CV-SF-614Gm) in which, in order to stabilize the spike protein structure, the 614G variant of the spike protein is used as a backbone and additionally 986(K) and 987(V) are substituted with proline and/or RRAR, which is a 682$^{nd}$ to 685$^{th}$ amino acid sequence, is mutated to QQAQ, but the present invention is not limited thereto.

In addition, sequence optimization was performed for the purpose of stabilizing mRNA and increasing translation efficiency in humans by increasing the amounts of guanine and cytosine in mRNA encoding the S antigen of SARS-CoV-2 virus, and among indicators that are able to predict RNA stability in detail, RNA fold, RNA fold thermodynamic ensemble, RNA structure, and cofold were confirmed for thermodynamic energy, and based on results thereof, CV-SF-WT-614D (mRNA encoding spike protein of SARS-CoV-2, SEQ ID NO: 1) and CV-SF-614Gm (mRNA encoding spike protein of SARS-CoV-2 614G variant, SEQ ID NO: 2), having the lowest ΔG value, were selected (Table 1).

[Table 1]

| | Sequence |
|---|---|
| CV-SF-WT-614D mRNA (SEQ ID NO: 1) | *AUGUUCGUGUUCCUGGUGCUGCUGCCUCUGGUGUCCAGCCAGUGUGUGAACC UGACCACCAGAACACAGCUGCCUCCAGCCUACACCAACAGCUUUACCAGAGG CGUGUACUACCCCGACAAGGUGUUCAGAUCCAGCGUGCUGCACUCUACCCAG GACCUGUUCCUGCCUUUCUUCAGCAACGUGACCUGGUUCCACGCCAUCCACG UGUCCGGCACCAAUGGCACCAAGAGAUUCGACAACCCCGUGCUGCCCUUCAA CGACGGGGUGUACUUUGCCAGCACCGAGAAGUCCAACAUCAUCAGAGGCUGG AUCUUCGGCACCACACUGGACAGCAAGACCCAGAGCCUGCUGAUCGUGAACA ACGCCACCAACGUGGUCAUCAAAGUGUGCGAGUUCCAGUUCUGCAACGACCC CUUCCUGGGCGUCUACUACCACAAGAACAACAAGAGCUGGAUGGAAAGCGAG UUCCGGGUGUACAGCAGCGCCAACAACUGCACCUUCGAGUACGUGUCCCAGC CUUUCCUGAUGGACCUGGAAGGCAAGCAGGGCAACUUCAAGAACCUGCGCGA GUUCGUGUUUAAGAACAUCGACGGCUACUUCAAGAUCUACAGCAAGCACACC CCUAUCAACCUCGUGCGGGAUCUGCCUCAGGGCUUCUCUGCUCUGGAACCCC UGGUGGAUCUGCCCAUCGGCAUCAACAUCACCCGGUUUCAGACACUGCUGGC CCUGCACAGAAGCUACCUGACACCUGGCGAUAGCAGCAGCGGAUGGACAGCU GGUGCCGCCGCUUACUAUGUGGGCUACCUGCAGCCUAGAACCUUCCUGCUGA AGUACAACGAGAACGGCACCAUCACCGACGCCGUGGAUUGUGCUCUGGAUCC UCUGAGCGAGACAAAGUGCACCCUGAAGUCCUUCACCGUGGAAAAGGGCAUC UACCAGACCAGCAACUUCCGGGUGCAGCCCACCGAAUCCAUCGUGCGGUUCC CCAAUAUCACCAAUCUGUGCCCCUUCGGCGAGGUGUUCAAUGCCACCAGAUU CGCCUCUGUGUACGCCUGGAACCGGAAGCGGAUCAGCAAUUGCGUGGCCGAC UACUCCGUGCUGUACAACUCCGCCAGCUUCAGCACCUUCAAGUGCUACGGCG UGUCCCCUACCAAGCUGAACGACCUGUGCUUCACAAACGUGUACGCCGACAG CUUCGUGAUCCGGGGAGAUGAAGUGCGGCAGAUUGCCCCUGGACAGACAGGC AAGAUCGCCGACUACAACUACAAGCUGCCCGACGACUUCACCGGCUGUGUGA UUGCCUGGAACAGCAACAACCUGGACUCCAAAGUCGGCGGCAACUACAAUUA CCUGUACCGGCUGUUCCGGAAGUCCAAUCUGAAGCCCUUCGAGCGGGACAUC UCCACCGAGAUCUAUCAGGCCGGCAGCACCCCUUGUAACGGCGUGGAAGGCU UCAACUGCUACUUCCCACUGCAGUCCUACGGCUUUCAGCCCACAAAUGGCGU GGGCUAUCAGCCCUACAGAGUGGUGGUGCUGAGCUUCGAACUGCUGCAUGCC CCUGCCACAGUGUGCGGCCCUAAGAAAAGCACCAAUCUCGUGAAGAACAAAU* |

(continued)

| | Sequence |
|---|---|
| | *GCGUGAACUUCAACUUCAACGGCCUGACCGGCACCGGCGUGCUGACAGAGAGCAACAAGAAGUUCCUGCCAUUCCAGCAGUUUGGCCGGGAUAUCGCCGAUACCACAGACGCCGUUAGAGAUCCCCAGACACUGGAAAUCCUGGACAUCACCCCUUGCAGCUUCGGCGGAGUGUCUGUGAUCACCCCUGGCACCAACACCAGCAAUCAGGUGGCAGUGCUGUACCAGGACGUGAACUGUACCGAAGUGCCCGUGGCCAUUCACGCCGAUCAGCUGACACCUACAUGGCGGGUGUACUCCACCGGCAGCAAUGUGUUUCAGACCAGAGCCGGCUGUCUGAUCGGAGCCGAGCACGUGAACAAUAGCUACGAGUGCGACAUCCCCAUCGGCGCUGGCAUCUGUGCCAGCUACCAGACACAGACAAACAGCCCUCAGCAGGCCCAGUCUGUGGCCAGCCAGAGCAUCAUUGCCUACACAAUGUCUCUGGGCGCCGAGAACAGCGUGGCCUACUCCAACAACUCUAUCGCUAUCCCCACCAACUUCACCAUCAGCGUGACCACAGAGAUCCUGCCUGUGUCCAUGACCAAGACCAGCGUGGACUGCACCAUGUACAUCUGCGGCGAUUCCACCGAGUGCUCCAACCUGCUGCUGCAGUACGGCAGCUUCUGCACCCAGCUGAAUAGAGCCCUGACAGGGAUCGCCGUGGAACAGGACAAGAACACCCAAGAGGUGUUCGCCCAAGUGAAGCAGAUCUACAAGACCCCUCCUAUCAAGGACUUCGGCGGCUUCAAUUUCAGCCAGAUUCUGCCCGAUCCUAGCAAGCCCAGCAAGCGGAGCUUCAUCGAGGACCUGCUGUUCAACAAAGUGACACUGGCCGACGCCGGCUUCAUCAAGCAGUAUGGCGAUUGUCUGGGCGACAUUGCCGCCAGGGAUCUGAUUUGCGCCCAGAAGUUUAACGGACUGACAGUGCUGCCUCCUCUGCUGACCGAUGAGAUGAUCGCCCAGUACACAUCUGCCCUGCUGGCCGGCACAAUCACAAGCGGCUGGACAUUUGGAGCUGGCGCCGCUCUGCAGAUCCCCUUUGCUAUGCAGAUGGCCUACCGGUUCAACGGCAUCGGAGUGACCCAGAAUGUGCUGUACGAGAACCAGAAGCUGAUCGCCAACCAGUUCAACAGCGCCAUCGGCAAGAUCCAGGACAGCCUGAGCAGCACAGCAAGCGCCCUGGGAAAGCUGCAGGACGUGGUCAACCAGAAUGCCCAGGCACUGAACACCCUGGUCAAGCAGCUGUCCUCCAACUUCGGCGCCAUCAGCUCUGUGCUGAACGAUAUCCUGAGCAGACUGGACCCUCCUGAGGCCGAGGUGCAGAUCGACAGACUGAUCACAGGCAGACUGCAGAGCCUCCAGACAUACGUGACCCAGCAGCUGAUCAGAGCCGCCGAGAUUAGAGCCUCUGCCAAUCUGGCCGCCACCAAGAUGUCUGAGUGUGUGCUGGGCCAGAGCAAGAGAGUGGACUUUUGCGGCAAGGGCUACCACCUGAUGAGCUUCCCUCAGUCUGCCCCUCACGGCGUGGUGUUUCUGCACGUGACAUAUGUGCCCGCUCAAGAGAAGAAUUUCACCACCGCUCCAGCCAUCUGCCACGACGGCAAAGCCCACUUUCCUAGAGAAGGCGUGUUCGUGUCCAACGGCACCCAUUGGUUCGUGACACAGCGGAACUUCUACGAGCCCCAGAUCAUCACCACCGACAACACCUUCGUGUCUGGCAACUGCGACGUCGUGAUCGGCAUUGUGAACAAUACCGUGUACGACCCUCUGCAGCCCGAGCUGGACAGCUUCAAAGAGGAACUGGACAAGUACUUUAAGAACCACACAAGCCCCGACGUGGACCUGGGCGAUAUCAGCGGAAUCAAUGCCAGCGUCGUGAACAUCCAGAAAGAGAUCGACCGGCUGAACGAGGUGGCCAAGAAUCUGAACGAGAGCCUGAUCGACCUGCAAGAACUGGGGAAGUACGAGCAGUACAUCAAGUGGCCCUGGUACAUCUGGCUGGGCUUUAUCGCCGGACUGAUUGCCAUCGUGAUGGUCACAAUCAUGCUGUGUUGCAUGACCAGCUGCUGUAGCUGCCUGAAGGGCUGUUGUAGCUGUGGCAGCUGCUGCAAGUUCGACGAGGACGAUUCUGAGCCCGUGCUGAAGGGCGUGAAACUGCACUACACCUGA* |

(continued)

| | Sequence |
|---|---|
| CV-SF-WT-614D protein (SEQ ID NO: 5) | *MFVFLVLLPLVSSQCVNLTTRTQLPPAYTNSFTRGVYYPDKVFRSSVLHSTQ DLFLPFFSNVTWFHAIHVSGTNGTKRFDNPVLPFNDGVYFASTEKSNIIRGW IFGTTLDSKTQSLLIVNNATNVVIKVCEFQFCNDPFLGVYYHKNNKSWMESE FRVYSSANNCTFEYVSQPFLMDLEGKQGNFKNLREFVFKNIDGYFKIYSKHT PINLVRDLPQGFSALEPLVDLPIGINITRFQTLLALHRSYLTPGDSSSGWTA GAAAYYVGYLQPRTFLLKYNENGTITDAVDCALDPLSETKCTLKSFTVEKGI YQTSNFRVQPTESIVRFPNITNLCPFGEVFNATRFASVYAWNRKRISNCVAD YSVLYNSASFSTFKCYGVSPTKLNDLCFTNVYADSFVIRGDEVRQIAPGQTG KIADYNYKLPDDFTGCVIAWNSNNLDSKVGGNYNYLYRLFRKSNLKPFERDI STEIYQAGSTPCNGVEGFNCYFPLQSYGFQPTNGVGYQPYRVVVLSFELLHA PATVCGPKKSTNLVKNKCVNFNFNGLTGTGVLTESNKKFLPFQQFGRDIADT TDAVRDPQTLEILDITPCSFGGVSVITPGTNTSNQVAVLYQDVNCTEVPVAI HADQLTPTWRVYSTGSNVFQTRAGCLIGAEHVNNSYECDIPIGAGICASYQT QTNSPQQAQSVASQSIIAYTMSLGAENSVAYSNNSIAIPTNFTISVTTEILP VSMTKTSVDCTMYICGDSTECSNLLLQYGSFCTQLNRALTGIAVEQDKNTQE VFAQVKQIYKTPPIKDFGGFNFSQILPDPSKPSKRSFIEDLLFNKVTLADAG FIKQYGDCLGDIAARDLICAQKFNGLTVLPPLLTDEMIAQYTSALLAGTITS GWTFGAGAALQIPFAMQMAYRFNGIGVTQNVLYENQKLIANQFNSAIGKIQD SLSSTASALGKLQDVVNQNAQALNTLVKQLSSNFGAISSVLNDILSRLDPPE AEVQIDRLITGRLQSLQTYVTQQLIRAAEIRASANLAATKMSECVLGQSKRV DFCGKGYHLMSFPQSAPHGVVFLHVTYVPAQEKNFTTAPAICHDGKAHFPRE GVFVSNGTHWFVTQRNFYEPQIITTDNTFVSGNCDVVIGIVNNTVYDPLQPE LDSFKEELDKYFKNHTSPDVDLGDISGINASVVNIQKEIDRLNEVAKNLNES LIDLQELGKYEQYIKWPWYIWLGFIAGLIAIVMVTIMLCCMTSCCSCLKGCC SCGSCCKFDEDDSEPVLKGVKLHYT* |

(continued)

| | Sequence |
|---|---|
| CV-SF-614Gm mRNA (SEQ ID NO: 2) | *AUGUUCGUGUUCCUGGUGCUGCUGCCUCUGGUGUCCAGCCAGUGUGUGAACC UGACCACCAGAACACAGCUGCCUCCAGCCUACACCAACAGCUUUACCAGAGG CGUGUACUACCCCGACAAGGUGUUCAGAUCCAGCGUGCUGCACUCUACCCAG GACCUGUUCCUGCCUUUCUUCAGCAACGUGACCUGGUUCCACGCCAUCCACG UGUCCGGCACCAAUGGCACCAAGAGAUUCGACAACCCCGUGCUGCCCUUCAA CGACGGGGUGUACUUUGCCAGCACCGAGAAGUCCAACAUCAUCAGAGGCUGG AUCUUCGGCACCACACUGGACAGCAAGACCCAGAGCCUGCUGAUCGUGAACA ACGCCACCAACGUGGUCAUCAAAGUGUGCGAGUUCCAGUUCUGCAACGACCC CUUCCUGGGCGUCUACUACCACAAGAACAACAAGAGCUGGAUGGAAAGCGAG UUCCGGGUGUACAGCAGCGCCAACAACUGCACCUUCGAGUACGUGUCCCAGC CUUUCCUGAUGGACCUGGAAGGCAAGCAGGGCAACUUCAAGAACCUGCGCGA GUUCGUGUUUAAGAACAUCGACGGCUACUUCAAGAUCUACAGCAAGCACACC CCUAUCAACCUCGUGCGGGAUCUGCCUCAGGGCUUCUCUGCUCUGGAACCCC UGGUGGAUCUGCCCAUCGGCAUCAACAUCACCCGGUUUCAGACACUGCUGGC CCUGCACAGAAGCUACCUGACACCUGGCGAUAGCAGCAGCGGAUGGACAGCU GGUGCCGCCGCUUACUAUGUGGGCUACCUGCAGCCUAGAACCUUCCUGCUGA AGUACAACGAGAACGGCACCAUCACCGACGCCGUGGAUUGUGCUCUGGAUCC UCUGAGCGAGACAAAGUGCACCCUGAAGUCCUUCACCGUGGAAAAGGGCAUC UACCAGACCAGCAACUUCCGGGUGCAGCCCACCGAAUCCAUCGUGCGGUUCC CCAAUAUCACCAAUCUGUGCCCCUUCGGCGAGGUGUUCAAUGCCACCAGAUU CGCCUCUGUGUACGCCUGGAACCGGAAGCGGAUCAGCAAUUGCGUGGCCGAC UACUCCGUGCUGUACAACUCCGCCAGCUUCAGCACCUUCAAGUGCUACGGCG UGUCCCCUACCAAGCUGAACGACCUGUGCUUCACAAACGUGUACGCCGACAG CUUCGUGAUCCGGGGAGAUGAAGUGCGGCAGAUUGCCCCUGGACAGACAGGC AAGAUCGCCGACUACAACUACAAGCUGCCCGACGACUUCACCGGCUGUGUGA UUGCCUGGAACAGCAACAACCUGGACUCCAAAGUCGGCGGCAACUACAAUUA CCUGUACCGGCUGUUCCGGAAGUCCAAUCUGAAGCCCUUCGAGCGGGACAUC UCCACCGAGAUCUAUCAGGCCGGCAGCACCCCUUGUAACGGCGUGGAAGGCU UCAACUGCUACUUCCCACUGCAGUCCUACGGCUUUCAGCCCACAAAUGGCGU GGGCUAUCAGCCCUACAGAGUGGUGGUGCUGAGCUUCGAACUGCUGCAUGCC CCUGCCACAGUGUGCGGCCCUAAGAAAAGCACCAAUCUCGUGAAGAACAAAU GCGUGAACUUCAACUUCAACGGCCUGACCGGCACCGGCGUGCUGACAGAGAG CAACAAGAAGUUCCUGCCAUUCCAGCAGUUUGGCCGGGAUAUCGCCGAUACC ACAGACGCCGUUAGAGAUCCCCAGACACUGGAAAUCCUGGACAUCACCCCUU GCAGCUUCGGCGGAGUGUCUGUGAUCACCCCUGGCACCAACACCAGCAAUCA GGUGGCAGUGCUGUACCAGGGCGUGAACUGUACAGAGGUGCCAGUGGCCAUU* |

(continued)

| | Sequence |
|---|---|
| | CACGCCGAUCAGCUGACCCCUACUUGGCGGGUGUACUCCACAGGCAGCAAUG<br>UGUUUCAGACCAGAGCCGGCUGUCUGAUCGGAGCCGAGCACGUGAACAAUAG<br>CUACGAGUGCGACAUCCCCAUCGGCGCUGGCAUCUGUGCCAGCUACCAGACA<br>CAGACAAACAGCCCUCAGCAGGCCCAGUCUGUGGCCAGCCAGAGCAUCAUUG<br>CCUACACAAUGUCUCUGGGCGCCGAGAACAGCGUGGCCUACUCCAACAACUC<br>UAUCGCUAUCCCCACCAACUUCACCAUCAGCGUGACCACAGAGAUCCUGCCU<br>GUGUCCAUGACCAAGACCAGCGUGGACUGCACCAUGUACAUCUGCGGCGAUU<br>CCACCGAGUGCUCCAACCUGCUGCUGCAGUACGGCAGCUUCUGCACCCAGCU<br>GAAUAGAGCCCUGACAGGGAUCGCCGUGGAACAGGACAAGAACACCCAAGAG<br>GUGUUCGCCCAAGUGAAGCAGAUCUACAAGACCCCUCCUAUCAAGGACUUCG<br>GCGGCUUCAAUUUCAGCCAGAUUCUGCCCGAUCCUAGCAAGCCCAGCAAGCG<br>GAGCUUCAUCGAGGACCUGCUGUUCAACAAAGUGACACUGGCCGACGCCGGC<br>UUCAUCAAGCAGUAUGGCGAUUGUCUGGGCGACAUUGCCGCCAGGGAUCUGA<br>UUUGCGCCCAGAAGUUUAACGGACUGACAGUGCUGCCUCCUCUGCUGACCGA<br>UGAGAUGAUCGCCCAGUACACAUCUGCCCUGCUGGCCGGCACAAUCACAAGC<br>GGCUGGACAUUUGGAGCUGGCGCCGCUCUGCAGAUCCCCUUUGCUAUGCAGA<br>UGGCCUACCGGUUCAACGGCAUCGGAGUGACCCAGAAUGUGCUGUACGAGAA<br>CCAGAAGCUGAUCGCCAACCAGUUCAACAGCGCCAUCGGCAAGAUCCAGGAC<br>AGCCUGAGCAGCACAGCAAGCGCCCUGGGAAAGCUGCAGGACGUGGUCAACC<br>AGAAUGCCCAGGCACUGAACACCCUGGUCAAGCAGCUGUCCUCCAACUUCGG<br>CGCCAUCAGCUCUGUGCUGAACGAUAUCCUGAGCAGACUGGACCCUCCUGAG<br>GCCGAGGUGCAGAUCGACAGACUGAUCACAGGCAGACUGCAGAGCCUCCAGA<br>CAUACGUGACCCAGCAGCUGAUCAGAGCCGCCGAGAUUAGAGCCUCUGCCAA<br>UCUGGCCGCCACCAAGAUGUCUGAGUGUGUGCUGGGCCAGAGCAAGAGAGUG<br>GACUUUUGCGGCAAGGGCUACCACCUGAUGAGCUUCCCUCAGUCUGCACCAC<br>ACGGCGUGGUGUUUCUGCACGUGACCUACGUGCCCGCUCAAGAGAAGAAUUU<br>CACCACCGCUCCAGCCAUCUGCCACGACGGCAAAGCCCACUUUCCUAGAGAA<br>GGCGUGUUCGUGUCCAACGGCACCCAUUGGUUCGUGACACAGCGGAACUUCU<br>ACGAGCCCCAGAUCAUCACCACCGACAACACCUUCGUGUCUGGCAACUGCGA<br>CGUCGUGAUCGGCAUUGUGAACAAUACCGUGUACGACCCUCUGCAGCCCGAG<br>CUGGACAGCUUCAAAGAGGAACUGGACAAGUACUUUAAGAACCACACAAGCC<br>CCGACGUGGACCUGGGCGAUAUCAGCGGAAUCAAUGCCAGCGUCGUGAACAU<br>CCAGAAAGAGAUCGACCGGCUGAACGAGGUGGCCAAGAAUCUGAACGAGAGC<br>CUGAUCGACCUGCAAGAACUGGGGAAGUACGAGCAGUACAUCAAGUGGCCUU<br>GGUACAUCUGGCUGGGCUUUAUCGCCGGACUGAUUGCCAUCGUGAUGGUCAC<br>AAUCAUGCUGUGUUGCAUGACCAGCUGCUGUAGCUGCCUGAAGGGCUGUUGU<br>AGCUGUGGCAGCUGCUGCAAGUUCGACGAGGACGAUUCUGAGCCCGUGCUGA<br>AAGGCGUGAAGCUGCACUACACCUGA |

(continued)

| | Sequence |
|---|---|
| CV-SF-614Gm protein (SEQ ID NO: 6) | *MFVFLVLLPLVSSQCVNLTTRTQLPPAYTNSFTRGVYYPDKVFRSSVLHSTQ DLFLPFFSNVTWFHAIHVSGTNGTKRFDNPVLPFNDGVYFASTEKSNIIRGW IFGTTLDSKTQSLLIVNNATNVVIKVCEFQFCNDPFLGVYYHKNNKSWMESE FRVYSSANNCTFEYVSQPFLMDLEGKQGNFKNLREFVFKNIDGYFKIYSKHT PINLVRDLPQGFSALEPLVDLPIGINITRFQTLLALHRSYLTPGDSSSGWTA GAAAYYVGYLQPRTFLLKYNENGTITDAVDCALDPLSETKCTLKSFTVEKGI YQTSNFRVQPTESIVRFPNITNLCPFGEVFNATRFASVYAWNRKRISNCVAD YSVLYNSASFSTFKCYGVSPTKLNDLCFTNVYADSFVIRGDEVRQIAPGQTG KIADYNYKLPDDFTGCVIAWNSNNLDSKVGGNYNYLYRLFRKSNLKPFERDI STEIYQAGSTPCNGVEGFNCYFPLQSYGFQPTNGVGYQPYRVVVLSFELLHA PATVCGPKKSTNLVKNKCVNFNFNGLTGTGVLTESNKKFLPFQQFGRDIADT TDAVRDPQTLEILDITPCSFGGVSVITPGTNTSNQVAVLYQGVNCTEVPVAI HADQLTPTWRVYSTGSNVFQTRAGCLIGAEHVNNSYECDIPIGAGICASYQT QTNSPQQAQSVASQSIIAYTMSLGAENSVAYSNNSIAIPTNFTISVTTEILP VSMTKTSVDCTMYICGDSTECSNLLLQYGSFCTQLNRALTGIAVEQDKNTQE VFAQVKQIYKTPPIKDFGGFNFSQILPDPSKPSKRSFIEDLLFNKVTLADAG FIKQYGDCLGDIAARDLICAQKFNGLTVLPPLLTDEMIAQYTSALLAGTITS GWTFGAGAALQIPFAMQMAYRFNGIGVTQNVLYENQKLIANQFNSAIGKIQD SLSSTASALGKLQDVVNQNAQALNTLVKQLSSNFGAISSVLNDILSRLDPPE AEVQIDRLITGRLQSLQTYVTQQLIRAAEIRASANLAATKMSECVLGQSKRV DFCGKGYHLMSFPQSAPHGVVFLHVTYVPAQEKNFTTAPAICHDGKAHFPRE GVFVSNGTHWFVTQRNFYEPQIITTDNTFVSGNCDVVIGIVNNTVYDPLQPE LDSFKEELDKYFKNHTSPDVDLGDISGINASVVNIQKEIDRLNEVAKNLNES LIDLQELGKYEQYIKWPWYIWLGFIAGLIAIVMVTIMLCCMTSCCSCLKGCC SCGSCCKFDEDDSEPVLKGVKLHYT* |

The vaccine composition of the present invention may further comprise liposomes or lipid nanoparticles (LNPs), and mRNA encoding the S antigen of SARS-CoV-2 virus may be adsorbed to or associated with the outside of the liposomes or lipid nanoparticles, or encapsulated inside.

The liposomes or lipid nanoparticles comprised in the vaccine composition of the present invention comprise a cationic lipid, and preferably additionally comprise a neutral lipid.

The cationic lipid is preferably at least one selected from the group consisting of dimethyldioctadecylammonium bromide (DDA), C12-200, 1,2-dioleoyl-3-trimethylammonium propane (DOTAP), 3β-[N-(N',N'-dimethylaminoethane)carbamoyl cholesterol (DC-Chol), 1,2-dioleoyloxy-3-dimethylammonium propane (DODAP), 1,2-di-O-octadecenyl-3-trimethylammonium propane (DOTMA), 1,2-dimyristoleoyl-sn-glycero-3-ethylphosphocholine (14:1 Ethyl PC), 1-palmitoyl-2-oleoyl-sn-glycero-3-ethylphosphocholine (16:0-18:1 Ethyl PC), 1,2-dioleoyl-sn-glycero-3-ethylphosphocholine (18:1 Ethyl PC), 1,2-distearoyl-sn-glycero-3-ethylphosphocholine (18:0 Ethyl PC), 1,2-dipalmitoyl-sn-glycero-3-ethylphosphocholine (16:0 Ethyl PC), 1,2-dimyristoyl-sn-glycero-3-ethylphosphocholine (14:0 Ethyl PC), 1,2-dilauroyl-sn-glycero-3-ethylphosphocholine (12:0 Ethyl PC), N1-[2-((1S)-1-[(3-aminopropyl)amino]-4-[di(3-aminopropyl)amino]butylcarboxamido)ethyl]-3,4-di[oleyloxy]-benzamide (MVL5), 1,2-dimyristoyl-3-dimethylammonium-propane (14:0 DAP), 1,2-dipalmitoyl-3-dimethylammonium-propane (16:0 DAP), 1,2-distearoyl-3-dimethylammonium-propane (18:0 DAP), N-(4-carboxybenzyl)-N,N-dimethyl-2,3-bis(oleoyloxy)propan-1-aminium (DOBAQ), 1,2-stearoyl-3-trimethylammonium-propane (18:0 TAP), 1,2-dipalmitoyl-3-trimethylammonium-propane (16:0 TAP), 1,2-dimyristoyl-3-trimethylammonium-propane (14:0 TAP), and N4-cholesteryl-spermine (GL67), but is not limited thereto, and is most preferably C12-200 or 1,2-dioleoyl-3-trimethylammonium propane (DOTAP), but is not limited thereto.

Cationic liposomes are generally known to be toxic, but the vaccine composition according to the present invention is detoxified by mRNA adsorption (Filion M. C. & Phillips N. C., Biochimica et Biophysica Acta (BBA)-Biomembranes, 1329(2), 345-356. 1997).

The liposomes or lipid nanoparticles comprising the cationic lipid according to the present invention may additionally comprise a neutral lipid.

The neutral lipid may be selected from the group consisting of 1,2-dioleoyl-sn-glycero-3-phosphoethanolamine (DOPE), 1,2-dimyristoyl-sn-glycero-3-phosphatidylcholine (DMPC), 1,2-dioleoyl-sn-glycero-3-phosphocholine (DOPC),

1,2-dipalmitoyl-sn-glycero-3-phosphocholine (DPPC), 1,2-distearoyl-sn-glycero-3-phosphocholine (DSPC), 1,2-dilinoleoyl-sn-glycero-3-phosphocholine (DLPC), phosphatidylserine (PS), phosphoethanolamine (PE), phosphatidylglycerol (PG), phosphoric acid (PA), phosphatidylcholine (PC), DOPI (1,2-dioleoyl-sn-glycero-3-phospho-(1'-myo-inositol)), and DSPI (1,2-distearoyl-sn-glycero-3-phosphoinositol), but is not limited thereto, and is most preferably 1,2-dioleoyl-sn-glycero-3-phosphoethanolamine (DOPE), but is not limited thereto.

The liposomes or lipid nanoparticles according to the present invention may further comprise at least one delivery factor selected from the group consisting of protamine, albumin, transferrin, PTD (protein transduction domain), CPP (cell penetrating peptide), polyethylene glycol (PEG), pegylated lipid, metal ion-linked lipid, and macrophage targeting moiety.

In the present invention, "DOTAP (dioleoyl-3-trimethylammonium propane)", which exemplifies a preferred cationic lipid, is a cationic emulsifier having the structure of Chemical Formula 1 below and is used as a fabric softener, and recently is used as a delivery carrier for proteins, compounds, peptides, etc., including nucleic acid carriers that form liposomes or lipid nanoparticles.

[Chemical Formula 1]

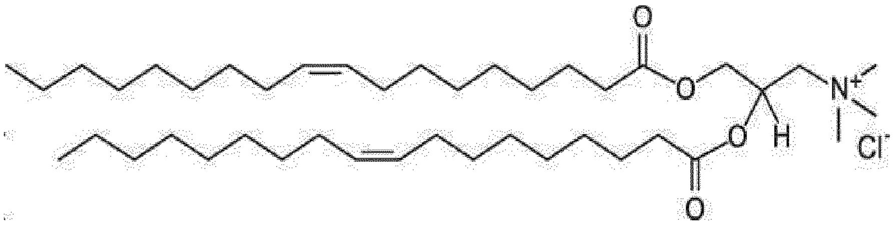

In addition, in the present invention, "DOPE (1,2-dioleoyl-sn-glycero-3-phosphoethanolamine)", which exemplifies a preferred neutral lipid, has the structure of Chemical Formula 2 below and is used as an auxiliary lipid for forming cationic liposomes or lipid nanoparticles.

[Chemical Formula 2]

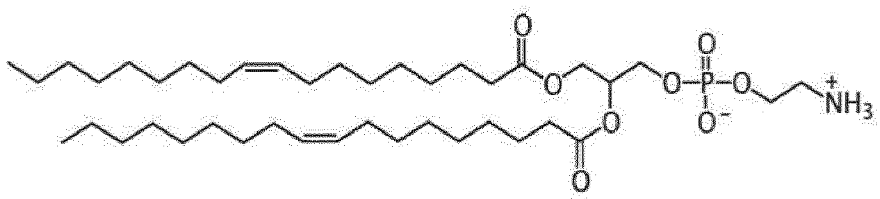

In the liposomes or lipid nanoparticles according to the present invention, the weight ratio of cationic lipid to neutral lipid is 1:9 to 9.5:0.5, preferably 2:8 to 9:1, more preferably 3:7 to 8:2, most preferably 4:6 to 7:3.

In the present invention, the liposomes or lipid nanoparticles according to the present invention may further comprise cholesterol. In the liposomes or lipid nanoparticles of the present invention, the weight ratio of cationic lipid to cholesterol is 6:1 to 1:3, preferably 4:1 to 1:2.5, more preferably 3:1 to 1:2, most preferably 2.5:1 to 1:1.5, but is not limited thereto.

Also, in the present invention, when the cationic lipid, neutral lipid, and cholesterol are all comprised in the liposomes or lipid nanoparticles according to the present invention, the weight ratio of cationic lipid to neutral lipid to cholesterol is 1-9.5:0.5-9:0.05-3, preferably 3-8:7-1:0.45-7.0, more preferably 1-3.5:1-3.5:0.5-3, but is not limited thereto.

In an embodiment of the present invention, the weight ratio of cationic lipid to neutral lipid to cholesterol was set to 2:2:1 (40:40:20, w/w/w), but is not limited thereto.

When cholesterol is additionally comprised, for example, when DOTAP and DOPE are used in a weight ratio of 1:1, cholesterol may be mixed in a weight ratio of 0.2-0.85, preferably 0.4-0.6 relative to DOTAP to form liposomes or lipid nanoparticles.

Also, in the vaccine composition for preventing SARS-CoV-2 according to the present invention, the mixing ratio of liposomes or lipid nanoparticles to mRNA may be represented as N:P ratio, and the N:P ratio affects mRNA expression and stability of the composition.

In the present invention, the N:P ratio of the liposomes or lipid nanoparticles to mRNA may be 0.2:1 to 1.4:1,

preferably 0.23:1 to 1.0:1, more preferably 0.46:1 to 1.0:1. In an exemplary embodiment of the present invention, the N:P ratio of 0.6:1 was used.

The vaccine composition of the present invention may further comprise an immune enhancer, but this is not essential, and a sufficient vaccine effect is exhibited even in the absence of an immune enhancer. The immune enhancer usable in the present invention is an immune enhancer selected from the group consisting of a material responding to a pattern recognition receptor (PRR) corresponding to a pathogen-associated molecular pattern (PAMP), CpG DNA, lipoprotein, flagella, poly I:C, saponin, squalene, tricaprin, 3D-MPL, and detoxified lipooligosaccharide (dLOS), but is not limited thereto.

In the immune enhancer, the detoxified lipooligosaccharide (dLOS) may be a material disclosed in Korean Patent No. 1509456 or Korean Patent No. 2042993, but is not limited thereto.

As used herein, the term "lipid nanoparticles", also called LNPs, refer to particles having at least one size on the order of nanometers (e.g. 1 to 1,000 nm) including one or more lipids. In some embodiments, such lipid nanoparticles include a cationic lipid and at least one excipient selected from among neutral lipids, charged lipids, steroids, and polymer conjugated lipids. In some embodiments, mRNA or a portion thereof is encapsulated in the lipid portion of lipid nanoparticles, or in an aqueous space surrounded by some or all of the lipid portion of lipid nanoparticles, and is thus protected from enzymatic degradation or other undesirable effects induced by mechanisms of the host organism or cell, such as negative immune responses. In some embodiments, mRNA or a portion thereof is associated with the lipid nanoparticles.

In the context of the present invention, the lipid nanoparticles are not limited to any particular form, and have to be interpreted to include any form that results when a cationic lipid or ionic lipid, and optionally at least one additional lipid, are combined in an aqueous environment and/or in the presence of a nucleic acid compound. For example, liposomes, lipid complexes, lipoplexes, etc. fall within the scope of lipid nanoparticles.

In various embodiments, the lipid nanoparticles have an average diameter of about 30 nm to about 400 nm, about 50 nm to about 400 nm, about 70 nm to about 400 nm, about 90 nm to about 400 nm, about 110 nm to about 400 nm, about 130 nm to about 400 nm, about 150 nm to about 400 nm, about 200 nm to about 400 nm, about 250 nm to about 400 nm, about 300 nm to about 400 nm, about 350 nm to about 400 nm, about 70 to about 90 nm, about 80 nm to about 90 nm, about 70 nm to about 80 nm, or about 30 nm, 40 nm, 50 nm, 60 nm, 70 nm, 80 nm, 90 nm, 100 nm, 110 nm, 120 nm, 130 nm, 140 nm, 150 nm, 160 nm, 170 nm, 180 nm, 190 nm, 200 nm, 210 nm, 220 nm, 230 nm, 240 nm, 250 nm, 260 nm, 270 nm, 280 nm, 290 nm, 300 nm, 310 nm, 320 nm, 330 nm, 340 nm, 350 nm, 360 nm, 370 nm, 380 nm, 390 nm, or 400 nm, and are substantially non-toxic. In some embodiments, mRNA resists degradation by nuclease in an aqueous solution when present in lipid nanoparticles. As used herein, the average diameter may be represented as a z-average value determined by dynamic light scattering.

The LNPs may include any lipids capable of forming particles to which one or more nucleic acid molecules are attached or in which one or more nucleic acid molecules are encapsulated. The term "lipid" refers to a group of organic compounds that are derivatives of fatty acids (e.g. esters) and are generally characterized by being insoluble in water but soluble in many organic solvents. Lipids are usually divided into at least three classes: (1) "simple lipids" including fats and oils as well as waxes; (2) "compound lipids" including phospholipids and glycolipids; and (3) "derived lipids" such as steroids.

The LNPs including mRNA comprise at least one cationic lipid and at least one stabilizing lipid as defined herein. The stabilizing lipid includes a neutral lipid and a pegylated lipid.

The LNPs include a cationic lipid. The cationic lipid is preferably cationizable. Specifically, the cationic lipid becomes protonated as the pH is lowered below the pKa of the ionizable group of the lipid, but becomes progressively more neutral at higher pH values. When positively charged, the lipid may be associated with a negatively charged nucleic acid. In some embodiments, the cationic lipid includes a zwitterionic lipid that becomes positively charged upon decreasing pH. The LNPs may include any lipid capable of forming particles to which one or more nucleic acid molecules are attached or in which one or more nucleic acid molecules are encapsulated.

In some embodiments, the LNPs may include any additional cationic or cationizable lipid, particularly any of a number of lipid species that possess a net positive charge at a selective pH, such as physiological pH.

In addition, the present invention provides a method of preparing the vaccine composition for preventing SARS-CoV-2 according to the present invention.

The method of preparing the vaccine composition for preventing SARS-CoV-2 according to the present invention may comprise adding a solution or buffer comprising liposomes or lipid nanoparticles to mRNA encoding the S antigen of SARS-CoV-2 virus or a solution or buffer comprising the same.

Here, mRNA encoding the S antigen of SARS-CoV-2 virus, or liposomes or lipid nanoparticles may be provided in the form of a lyophilized powder or in a state of being dissolved in an appropriate solution or buffer. When mRNA encoding the S antigen of SARS-CoV-2 virus or liposomes or lipid nanoparticles are provided in a lyophilized state, they are dissolved in an appropriate solution or buffer, and the solution or buffer comprising liposomes or lipid nanoparticles may be added to mRNA encoding the S antigen of SARS-CoV-2 virus or the solution or buffer comprising the same, thereby preparing a vaccine composition for preventing SARS-CoV-2 according to the present invention.

Also, when dLOS is further included in the vaccine composition encoding the S antigen of SARS-CoV-2 virus according to the present invention, mRNA encoding the S antigen of SARS-CoV-2 virus or a solution or buffer comprising the same may be added to dLOS or a solution or buffer comprising the same, and a solution or buffer comprising liposomes or lipid nanoparticles may be added thereto, thereby preparing a vaccine composition for preventing SARS-CoV-2 according to the present invention.

dLOS may be provided in the form of a lyophilized powder or in a state of being dissolved in an appropriate solution or buffer. When dLOS is provided in a lyophilized state, it may be used after being dissolved in an appropriate solution or buffer.

In an embodiment of the present invention, F-EG-COVID, which is a lyophilized formulation of EG-COVID as a vaccine for preventing SARS-CoV-2 according to the present invention, was confirmed to exhibit superior immunogenicity even after storage for 8 weeks at -2 to 8°C (FIG. 7).

Cationic liposomes used in the present invention are known to have a depot effect (Therapeutic Advances in Vaccines 2(6) : 159-82) . In another embodiment of the present invention, EG-COVID, which is a vaccine for preventing SARS-CoV-2 using cationic liposomes as a delivery carrier, was administered to the left thigh muscle of rats, and the expression of CV-SF-614G mRNA in the serum and each tissue of rats was confirmed over time, and also CV-SF-614G mRNA was confirmed to be expressed only at the site of administration even after lapse of time (FIG. 8).

In still another embodiment of the present invention, based on results of measurement of the SARS-CoV-2 infection inhibitory effect in Vero 76 cells using the serum of mice administered with EG-COVID, which is a vaccine for preventing SARS-CoV-2, the infection inhibitory effect was confirmed to increase with an increase in the amount of mRNA of EG-COVID (FIG. 9A).

In yet another embodiment of the present invention, EG-COVID of the present invention was confirmed to exhibit superior cross-immunity effect against Alpha and Beta variants as SARS-CoV-2 variants (FIG. 9B).

Another aspect of the present invention pertains to a method of preventing SARS-CoV-2 infection comprising administering a composition for preventing SARS-CoV-2 comprising mRNA encoding the S antigen of SARS-CoV-2 virus.

Still another aspect of the present invention pertains to the use of the composition comprising mRNA encoding the S antigen of SARS-CoV-2 virus for the prevention of SARS-CoV-2 infection.

Yet another aspect of the present invention pertains to the use of the composition for preventing SARS-CoV-2 comprising mRNA encoding the S antigen of SARS-CoV-2 virus for the manufacture of a medicament for the prevention of SARS-CoV-2 infection.

## Examples

A better understanding of the present invention may be obtained through the following examples. These examples are merely set forth to illustrate the present invention, and are not to be construed as limiting the scope of the present invention, as will be apparent to those skilled in the art.

### Example 1: Selection of mRNA sequences

The mRNA sequence was used to encode a sequence (CV-SF-614Gm) in which, in order to stabilize the spike protein structure, 986(K) and 987(V) were substituted with proline and RRAR, which is a $682^{nd}$ to $685^{th}$ amino acid sequence, was mutated to QQAQ.

In sequences for the spike protein of SARS-CoV-2 and the spike protein of SARS-CoV-2 614G variant, sequence optimization was performed using the following three programs for the purpose of stabilizing mRNA and increasing translation efficiency in humans by increasing the amounts of guanine and cytosine in mRNA.

Program 1: GenSmart codon optimization program (Genscript)
Program 2: Integrated DNA technologies codon optimization program (IDT, Integrated DNA Technologies)
Program 3: GenArt codon optimization program

(Thermo Fisher)

Among indicators capable of predicting RNA stability in detail, RNA fold, RNA fold thermodynamic ensemble, RNA structure, and cofold were confirmed for thermodynamic energy. It is generally known that the lower the $\Delta G$, the higher the thermodynamic stability, and thus CV-SF-WT-614D (mRNA encoding spike protein of SARS-CoV-2, SEQ ID NO: 1) and CV-SF-614Gm (mRNA encoding spike protein of SARS-CoV-2 614G variant, SEQ ID NO: 2) were selected as sequences obtained through Program 3 with the lowest $\Delta G$ value.

The selected mRNA sequences were synthesized through *in-vitro* transcription by TriLink BioTechnologies.

The DNA sequences of CV-SF-WT-614D and CV-SF-614Gm are represented by SEQ ID NO: 3 and SEQ ID

NO: 4, respectively, and the amino acid sequences of CV-SF-WT-614D and CV-SF-614Gm are represented by SEQ ID NO: 5 and SEQ ID NO: 6, respectively.

[Table 2]

| Candidate | | Unoptimized sequence | Program 1 | Program 2 | Program 3 |
|---|---|---|---|---|---|
| CV-SF-WT-614D | RNA fold | -1068.90 | -1254.70 | -1122.90 | **-1328.70** |
| | RNA fold thermodynamic ensemble | -1133.67 | -1315.84 | -1188.67 | **-1386.34** |
| | RNA structure | -1076.30 | -1270.80 | -1134.90 | **-1341.50** |
| | Cofold | -1050.07 | -1257.37 | -1127.66 | **-1334.45** |
| CV-SF-614Gm | RNA fold | -1073.00 | -1283.20 | -1128.50 | **-1331.00** |
| | RNA fold thermodynamic ensemble | -1137.55 | -1342.44 | -1193.18 | **-1386.79** |
| | RNA structure | -1080.40 | -1292.50 | -1143.30 | **-1341.50** |
| | Cofold | -1066.02 | -1275.11 | -1141.76 | **-1324.42** |

**Example 2: Preparation of liposome delivery carrier using film method**

Each of DOTAP (Merck & Cie/CH2900014), DOPE (Avanti Polar Lipid), and cholesterol (Avanti Polar Lipid) was mixed with chloroform in a ratio of 40:40:20 and completely dissolved at 37°C for 10 minutes to form liquid solutions.
A lipid mixture was prepared by mixing the liquid solutions at a predetermined weight ratio in a round bottom flask, and the lipid mixture containing DOTAP was volatilized at 60°C for 30 minutes in a rotary evaporator (Buchi/B491_R200) to remove chloroform, and a lipid membrane film was formed on the wall of the flask.
20 mM HEPES buffer (pH 7.4) containing 4% (w/v) sucrose was placed in the flask, and the lipid membrane was dissolved at 60°C to form liposomes. The liposomes thus formed were measured for particle size, zeta potential, and dispersity using a dynamic light scattering analyzer. The remaining prepared liposomes were stored in a refrigerator at 4°C until testing.

**Example 3: Confirmation of i*n-vitro* expression depending on lipid composition of liposomes**

For liposomes, it has been reported that, when phosphatidylcholine (hereinafter referred to as 'PC')-type lipids are changed to phosphoethanolamine (hereinafter referred to as 'PE')-type lipids, the expression level of erythropoietin (hereinafter referred to as 'EPO') protein is increased about 7 times (Dowhan W et al., New Comprehensive Biochemistry, 36(4): 1-35, 2002; Leung A-KK et al., The Journal of Physical Chemistry B, 119(28): 8698-8706, 2015; Kauffman KJ et al., Nano letters, 15(11) :7300-7306, 2015).
Therefore, in order to search for the optimal liposome composition for increasing mRNA delivery efficiency, attempts were made to investigate optimal conditions capable of increasing mRNA delivery efficiency *in vivo* using liposomes of the following four compositions by adding cholesterol (Chol) to DOTAP and DMPC, which are constituent lipids of cationic liposomes applied conventionally to herpes zoster vaccine (EG-HZ), or replacing DMPC with 1,2-dioleoyl-sn-glycero-3-phosphoethanolamine (hereinafter referred to as 'DOPE').
First group: DOTAP/DMPC (50:50, w/w)
Second group: DOTAP/DMPC/Chol (40:40:20, w/w/w)
Third group: DOTAP/DOPE (50:50, w/w)
Fourth group: DOTAP/DOPE/Chol (40:40:20, w/w/w)

Liposomes composed of different lipids were prepared as above, 20 μg of Renilla luciferase (TriLink BioTechnologies) and 80 μg of liposomes were mixed, and then a total of 100 μL thereof was intramuscularly administered to 6-week-old female C57BL/6N mice [Japan SLC] (Central Lab. Animal Inc.) (n=4-5/group). Six hours after administration, the mice were anesthetized by intraperitoneal administration of 250 mg/kg of Avertin working solution, and 0.15 mg/mL of Renilla luciferase substrate solution was prepared using 2.4 mL of 1x PBS with 0.37 mg/vial Renilla luciferase substrate stock solution and then 1 mg/kg of Renilla luciferase substrate solution was administered intravenously to the tails of the mice. Immediately after administration, the mice were placed in an *in-vivo* imaging system (IVIS) (Ami HTX) and then photographed by setting the exposure time to 60 seconds, and the values of region of interest (hereinafter referred

to as 'ROI') of the site of administration were compared.

Thereby, as shown in FIG. 2, it was confirmed that the cationic liposomes including DOTAP/DOPE/Chol mixed in a ratio of 40:40:20 (w/w/w) had the highest delivery efficiency *in vivo,* which was named CV-LP-b1 and used as an mRNA delivery system for EG-COVID.

### Example 4: Analysis of properties of liposomes

For development of EG-COVID, which is a vaccine for preventing SARS-CoV-2 virus infection, physical properties of 3 batches of CV-LP-b1, which is the mRNA delivery system confirmed in Example 3, were analyzed.

CV-LP-b1 liposomes mixed in 20 mM HEPES buffer (pH 7.4) containing 4% sucrose were subjected to dynamic light scattering (DLS) analysis using Malvern/ZSP and the mean and standard deviation of particle size, dispersity, and zeta potential thereof were determined.

Thereby, CV-LP-b1 was measured to have an average particle size of 80.3±3.0 d. nm, dispersity of 0.194±0.010, and zeta potential of 50.7±3.0 mV.

Considering that the size of typical liposomes is 50-250 d. nm (Korean Patent Application Publication No. 2014-0097215), the size of the liposomes was at an appropriate level, and it was known that, when the zeta potential of the liposomes was 30 mV or higher in case of positive charges, aggregation did not occur and the structure was stably maintained (Antisense drug technology; Principles, Strategy, and Application, CRC press, second edition, 253, 2007), and the zeta potential of the liposomes was analyzed beyond that, assuming that the liposomes were electrostatically stable. Moreover, the PDI values of the liposomes were all 0.25 or less, confirming that the liposomes had a particle distribution close to mono-dispersity in a stable state (Appl. Chem. Eng., 28 (2): 177-185, 2017).

### Example 5: Confirmation of NP ratio

An mRNA-liposome complex was prepared by mixing and adsorbing the liposomes (LPs) prepared in Examples 1 and 2 and mRNA as main ingredients in 20 mM HEPES (pH 7.4) containing 4% sucrose.

The N/P ratio, which is the mixing ratio of liposomes and mRNA, was calculated using the following equation. The prepared complexes were the SEQ ID NO: 8 EGFP mRNA-liposome complex, the SEQ ID NO: 7 RLuc mRNA-liposome complex, and the SEQ ID NO: 2 CV-SF-614Gm mRNA-liposome complex, which were used for DLS, *in-vivo* expression, and immunogenicity experiments, respectively.

$$(N/P\ ratio) = \frac{mass\ of\ DOTAP\ (g)}{MW\ of\ DOTAP} : \frac{mass\ of\ mRNA\ (g)}{MW\ of\ mRNA} \times mRNA\ base\ pair$$

Each mRNA-liposome complex was prepared by varying the NP ratio of liposomes and mRNA forming a complex, and *in-vivo* expression of the mRNA-liposome complex was confirmed using mice.

100 μL of the mRNA complex expressing RLuc was injected intramuscularly into the thigh deltoid of 6-week-old female mice (C57BL/6N, Orient Bio, Central Lab. Animal Inc.).

Six hours after administration of the test material, the mice were anesthetized by intraperitoneal administration of 250 mg/kg of Avertin working solution, 0.15 mg/mL of Renilla luciferase substrate solution was prepared using 2.4 mL of 1x PBS and 0.37 mg/vial Renilla luciferase substrate stock solution (Promega), and then 1 mg/kg of Renilla luciferase substrate solution was administered intravenously to the tails of the mice. Immediately after administration, the mice were photographed by setting the exposure time to 60 seconds using Ami-HTX (Xenogen IVIS-200, Spectral Instruments Imaging, USA), and the expression level of the site of administration was quantified using Aura Imaging Software (Spectral Instruments Imaging, USA).

Thereby, as shown in FIG. 3, mRNA expression efficiency increased in mice when the NP ratio was 0.23 or higher, and the expression was the highest at 0.46:1 to 1.0:1. The mixing ratio of mRNA and liposomes was set to NP ratio = 0.6 in the following experiment.

### Example 6: *In-vitro* expression by selected liposomes

*In-vitro* expression was confirmed for a formulation in which CV-LP-b1 was mixed with CV-SF-614Gm (hereinafter referred to as 'EG-COVID') whether expression occurred normally.

HEK293T cells (Homo sapiens embryonic kidney 293T cell, CRL-3216/ATCC) were seeded at $2\times10^6$ cells/dish in a 100 mm dish, cultured overnight at 37°C in a $CO_2$ incubator, and transfected at 60-70% confluency.

For transfection of CV-SF-614Gm, the cells were treated with a mixture of lipofectamine 3000 (Thermo Fisher) and CV-LP-b1 for 24 hours at 37°C in a $CO_2$ incubator.

After 24 hours, all the cell culture media was removed, followed by washing with D-PBS, after which 400 μL of 1x cell lysis solution was dispensed into a 100 mm culture dish on ice, after which the cells were collected in a 1.7 mL tube using a scraper, vortexed, and then centrifuged at 15,000 rpm and 4°C for 15 minutes to collect protein in the supernatant. The protein thus collected was quantified, and 20 μg/well of protein was subjected to SDS-PAGE and Western blot analysis was performed.

Antibodies used for Western blot analysis are as follows.

SARS-CoV-2 antibody [NB100-56578/Novus biologicals/ab092903c-15, (immunogen; SARS-CoV-2, amino acid 1124-1140 from S2 protein)]
SARS-CoV-2 antibody [MBS434243/Mybiosource/T1218EL, (immunogen; SARS-CoV-2 S-full protein)]
SARS-CoV-2 antibody [40591-MM42/Sino biological/HA14AP3001, (immunogen; SARS-CoV-2 S1-mFC protein)
β-actin rabbit mAb HRP conjugate [5125/Cell signaling/6]
Goat anti-rabbit IgG (H+L) Cross-Adsorbed Secondary Antibody, HRP [G21234/Thermo/2087715]
Goat anti-mouse IgG secondary antibody, HRP [SSA007/Sino biological/HO21OC1101]

Thereby, as shown in FIG. 4A, it was confirmed that the expression depending on the concentration of CV-SF-614Gm using lipofectamine 3000 (Thermo Fisher) appeared in a concentration-dependent manner. Moreover, as shown in FIG. 4B, based on results of observation of expression by EG-COVID composed of different amounts of CV-SF-614Gm, it was confirmed that the S protein was normally expressed.

**Example 8: Confirmation of immunogenicity depending on dose of mRNA (CV-SF-614Gm)**

CV-LP-b1 mixed with different doses of mRNA was administered at 0.1 HD (human dose) to 6-week-old female B6C3F1/slc mice (Central Lab. Animal Inc.) twice at 3-week intervals, the mice were sacrificed 2 weeks after final immunization, the serum was isolated therefrom, and the end-point titer was determined by analyzing the SARS-CoV-2 receptor-binding domain (RBD) protein-specific total IgG antibody titer ($\log_{10}$) by indirect ELISA.

In order to confirm immunogenicity of the mRNA-liposome complex, 6-week-old female B6C3F1/slc mice (Japan SLC) were selected as target animals, CV-LP-b1 mixed with different doses of mRNA was administered at 0.1 HD (human dose) to the mice twice at 3-week intervals, the mice were sacrificed 2 weeks after final immunization, serum was isolated therefrom, and the end-point titer was determined by analyzing the SARS-CoV-2 receptor-binding domain (RBD) protein-specific total IgG antibody titer ($\log_{10}$) by indirect ELISA.

8-1: Blood collection

Two weeks after the last administration, mice were anesthetized by intraperitoneal administration of 250 mg/kg of Avertin working solution, and whole blood was collected through cardiac blood sampling. The whole blood thus collected was transferred to a microtube, allowed to stand at room temperature for 3 hours, and centrifuged at 4°C and 15,000 rpm for 10 minutes, after which the supernatant was transferred to a new microtube and serum was obtained and stored at -20°C until analysis.

8-2: Splenocyte restimulation

After the mice were sacrificed by cervical dislocation and the spleens were extracted therefrom, the spleens of each group were pooled and transferred to a 24-well plate into which PBS supplemented with 1% penicillin-streptomycin solution (hereinafter, PBS w/antibiotics) was dispensed. The media used are shown in Tables 3 and 4 below.

[Table 3]

| Complete media | Added amount | Final concentration |
|---|---|---|
| RPMI 1640 medium | 439.9 mL | - |
| FBS (Non-heat inactivated) | 50 mL | 10% (v/v) |
| Penicillin-streptomycin solution (100x) (Antibiotics) | 5 mL | Penicillin (100 U/mL), Streptomycin (100 μg/mL) |
| HEPES buffer solution (1 M) | 5 mL | 10 mM |
| 0.5 M β-mercaptoethanol | 50 μL | 50 μM |

(continued)

| Complete media | Added amount | Final concentration |
|---|---|---|
| Recombinant mouse IL-2 | 12.5 μL | 0.5 ng/mL |

[Table 4]

| Basal media | Added amount | Final concentration |
|---|---|---|
| RPMI 1640 medium | 439.9 mL | - |
| FBS (Non-heat inactivated) | 50 mL | 10% (v/v) |
| Penicillin-streptomycin | 5 mL | Penicillin (100 U/mL), |
| solution (100x) (Antibiotics) | | Streptomycin (100 μg/mL) |
| HEPES buffer solution (1 M) | 5 mL | 10 mM |

The spleen tissue was washed with PBS containing antibiotics in a clean bench, transferred to a 60 mm dish containing 3 mL of basal media, and crushed with a 40 um cell strainer to isolate splenocytes. The isolated splenocytes were transferred to a 15 mL tube and centrifuged at 4°C and 3,000 rpm for 5 minutes to remove the supernatant, and the splenocytes were treated with 3 mL of RBC lysis buffer (Thermo Fisher), allowed to stand at room temperature for 3 minutes, and then centrifuged at 4°C and 3,000 rpm for 5 minutes.

After the supernatant was removed, the cells were suspended in 3 mL of PBS containing antibiotics and centrifuged at 4°C and 3,000 rpm for 5 minutes to remove the supernatant, and the cells were suspended in 10 mL of complete media (Gibco).

The cell suspension was diluted to $2\times10^7$ cells/mL using complete media and then dispensed at 100 μL/well into a 96-well cell culture plate.

PepMix SARS-CoV-2-S1 peptide pool (JPT Peptide Technologies) and S2 peptide pool (JPT Peptide Technologies) were dissolved in 50 μL of DMSO in respective vials and then mixed with complete media to a final concentration of 2.5 μg/mL, thus preparing SARS-CoV-2 spike peptide stimulants.

40 μg/well of the stimulant and 60 μL/well of the complete media were added to 96 wells containing the cell suspension, followed by reaction at 37°C and 5% $CO_2$ for 72 hours.

8-3: Analysis of antibody titer

An RBD antigen (Mybiosource, USA) was diluted to 1 ug/mL with 1x PBS, after which 100 μL thereof was dispensed into an immunoplate, covered with a sealing film, and allowed to stand in a refrigerator at 4°C overnight. Wash buffer was prepared by diluting 20x PBS with purified water to obtain 1 L of 1x PBS and adding 500 μL of Tween 20 thereto. The solution in each well was removed with an ELISA washer (Tecan/Hydroflexelisa), followed by washing five times using wash buffer.

A reagent diluent (1% BSA) was prepared by dissolving 1 g of BSA in 100 mL of PBS, dispensed at 200 μL/well into the immunoplate, covered with a sealing film, and allowed to stand in an incubator at 37°C for 1 hour. The solution in each well was removed with an ELISA washer, followed by washing five times using wash buffer. The reagent diluent was dispensed at 100 μL/well into the immunoplate. The serum sample obtained by the method of 6-1 above was diluted 1:50 using the reagent diluent, 100 μL thereof was dispensed in line 1 of B to G of the immunoplate, and the sample was mixed by pipetting several times in the well. Thereafter, the sample was subjected to 1/2 serial dilution up to line 12 on the ELISA plate in a manner of taking 100 μL from line 1 and adding the same to line 2.

In order to evaluate adequacy of the test, hyperserum was diluted 1:200 using the reagent diluent, after which 100 μL thereof was dispensed in line 1 of H of the immunoplate, followed by 1/2 serial dilution in the same manner as above.

The immunoplate was covered with a sealing film, followed by reaction in an incubator at 37°C for 2 hours. The solution in each well was removed with an ELISA washer, followed by washing five times using wash buffer.

A goat anti-mouse IgG antibody (Jackson Laboratory) was diluted 1:5,000 using the reagent diluent, dispensed in an amount of 100 μL into an immunoplate, covered with a sealing film, and allowed to react in an incubator at 37°C for 1 hour.

The solution in each well was removed with an ELISA washer, followed by washing five times using wash buffer. 100 μL of TMB substrate solution equilibrated to room temperature was dispensed into an immunoplate and allowed to react for 5 minutes in the dark at room temperature. The reaction was stopped by dispensing 100 μL of 1 N

$H_2SO_4$ solution to the immunoplate, and absorbance was measured at 450 nm using an ELISA reader (Biotek/Epoch).

8-4: Cytokine assay (ELISA)

The following test was conducted using an IFN-γ ELISA kit (Mouse IFN-γ Duoset ELISA, R&D systems).
The splenocyte culture fluid stimulated by the splenocyte restimulation method of 6-2 above was diluted 1/5 with a reagent diluent, dispensed at 100 μL/well into a microplate coated with an anti-mouse IFN-γ capture antibody (Jackson), covered with a sealing film, and allowed to stand at room temperature for 2 hours, after which the solution in each well was removed with an ELISA washer (Tecan/Hydroflexelisa), followed by washing three times using wash buffer.
Streptavidin-HRP in the kit was diluted 1/40 using a reagent diluent, dispensed at 100 μL/well into an immunoplate, covered with a sealing film, and allowed to stand at room temperature for 20 minutes, after which the solution in each well was removed with an ELISA washer, followed by washing three times using wash buffer.
An anti-mouse IFN-γ detection antibody in the kit was diluted to 200 ng/mL using a reagent diluent, dispensed at 100 μL/well into an immunoplate, covered with a sealing film, and allowed to stand at room temperature for 1 hour, after which the solution in each well was removed with an ELISA washer (Tecan/Hydroflexelisa), followed by washing three times using wash buffer.
100 μL of TMB substrate (KPL SureBlue TMB microwell peroxidase substrate, Seracare) solution was dispensed into an immunoplate and allowed to react for 15 minutes in the dark at room temperature, after which the reaction was stopped by dispensing 100 μL of 1 N $H_2SO_4$ solution into the immunoplate, and absorbance was measured at 450 nm using an ELISA reader.

8-5: Confirmation of neutralization capacity

For serum samples obtained by administering the EG-COVID vaccine of the present invention, whether the vaccine effectively inhibits virus infection was evaluated using a SARS-CoV-2 surrogate virus neutralization test (sVNT) kit (Genscript).
60 μL of each of the negative control, positive control, and serum sample, and 60 μL of 1:1000 diluted-HRP conjugated RBD were mixed in a 1.5 mL microtube and then allowed to react in an incubator at 37°C for 30 minutes, after which 100 μL of the resulting reaction mixture was dispensed into a microtiter test strip plate, covered with a sealing film, and allowed to react in an incubator at 37°C for 15 minutes.
The solution in each well was removed with an ELISA washer (Tecan/Hydroflexelisa), followed by washing four times using 1x wash solution.
100 μL/well of TMB solution (Thermo Fisher) was dispensed, covered with a sealing film, and allowed to react for 15 minutes in the dark at room temperature, after which the reaction was stopped by dispensing 50 μL/well of stop solution, the optical density was measured at 405 nm using an ELISA reader (Thermo Scientific), and then the neutralization capacity (neutralization%) was determined as below.
FIG. 5A shows the RBD-specific IgG antibody titer analyzed by ELISA in the serum of mice administered with the mRNA complex in which CV-LP-b1 and CV-SF-614Gm were mixed, confirming superior delivery efficiency by adsorption up to 30 μg of mRNA. FIG. 5B shows the concentration of IFN-γ analyzed by ELISA in the serum of mice administered with the mRNA complex in which CV-LP-b1 and CV-SF-614Gm were mixed, indicating high IFN-γ concentration at 5 μg and 10 μg of mRNA. FIG. 5C shows the neutralization capacity of the obtained serum analyzed using a SARS-CoV-2 surrogate virus neutralization test (sVNT) kit, indicating high ability to induce immunity at 5 μg or more of mRNA.
When the above results were converted into HD (human dose), CV-LP-b1 was confirmed to exhibit superior delivery efficiency by adsorption up to 300 μg of mRNA, and 50 μg or more of mRNA was judged to be suitable for immunity induction. However, cellular immunity in view of IFN-gamma concentration was found to be vastly superior at 5 to 10 μg.

## Example 9: Difference in immunogenicity depending on frequency of administration

CV-LP-b1 was mixed with 200 ug of CV-SF-614Gm at NP ratio=0.6:1 and then lyophilized, after which a difference in immunogenicity upon administration once and twice using 0.1 HD thereof was confirmed.
6-week-old female B6C3F1/slc mice (Central Lab. Animal Inc.) were immunized twice at 3-week intervals and sacrificed 2 weeks later, or sacrificed 2 weeks after single administration, after which immunogenicity was confirmed using the obtained serum and splenocytes (n=6/group).
The test was performed in the same manner as in Example 8, and the results thereof are shown in FIG. 6, confirming that the immunogenicity, including humoral and cellular immunity, was superior when administered twice

rather than once.

**Example 10: Confirmation of stability of lyophilized formulation**

Currently developed mRNA-based vaccines, namely COVID-19 preventive vaccines from Pfizer and Moderna, use lipid nanoparticles (hereinafter referred to as 'LNPs') as mRNA delivery carriers, and thus distribution under cryogenic storage (-20°C to -80°C) is required due to poor refrigeration storage stability thereof.

On the other hand, EG-COVID uses cationic liposomes, and in the present example, L-EG-COVID, which is a liquid formulation of the cationic liposome CV-LP-b1 and CV-SF-614Gm complex (EG-COVID) refrigerated for 8 weeks, and F-EG-COVID, which is a lyophilized formulation, were measured for *in-vivo* efficacy and comparatively analyzed with immunogenicity of finished products refrigerated for 0 week and 4 weeks, whereby *in-vivo* efficacy and stability depending on the type of formulation and on the refrigeration storage period were compared in the same manner as in Example 8.

EG-COVID in the present example was formulated to include 100 μg of CV-SF-614Gm, and after storage of a liquid formulation or lyophilized formulation thereof at -2 to 8°C for a predetermined period of time, the lyophilized formulation was rehydrated and subjected to an immunogenicity test to confirm that the efficacy was maintained.

A lyophilized formulation was prepared from the liquid formulation of Example 2 by the following method.

0.65 mL of the liquid EG-COVID formulation was dispensed into 2 mL sterile glass vials, half-closed with rubber stoppers, transferred to a freeze dryer (Ilshin Biobase), and lyophilized in the order of -40°C (50 mTorr) for 10 hours, -20°C (50 mTorr) for 10 hours, and 20°C (50 mTorr) for 20 hours.

After completion of lyophilization, the vials were completely closed with the rubber stoppers, sealed, and stored at 2-8°C.

Thereby, as shown in FIG. 7, the liquid formulation was found to be unable to induce immunogenicity due to a drastic decrease in stability after 4 weeks of storage, but the lyophilized formulation stably maintained immunogenicity up to 8 weeks, confirming that the storage stability of the lyophilized formulation of EG-COVID was excellent.

**Example 11: Confirmation of biodistribution**

The COVID-19 preventive vaccine EG-COVID according to the present invention uses cationic liposomes as an mRNA delivery carrier in order to efficiently deliver CV-SF-614Gm into the body. In previous studies by the present inventors, based on the results of measurement of the expression pattern of luciferase protein *in vivo* over time after intramuscular administration of a mixture of Renilla luciferase-encoding mRNA and CV-LP-b1 to mice, it was confirmed that the protein was expressed only at the site of administration and not expressed in other organs from 6 hours to 24 hours after administration.

In the present example, EG-COVID was intramuscularly administered to rats, after which the distribution of CV-SF-614Gm was observed in the site of administration and the major organs other than the site of administration over time. To this end, the relative value of CV-SF-614Gm to GAPDH, which is a housekeeping gene, was measured using RT-qPCR to confirm the biodistribution pattern of CV-SF-615Gm after EG-COVID administration.

In order to confirm the biodistribution pattern of CV-SF-614Gm over time, EG-COVID was administered to the left thigh muscle of rats, and after 0, 2, 6, 24, 48, 72, and 120 hours, the presence of CV-SF-614Gm *in vivo* was confirmed through RT-qPCR.

As test animals, 105 6-week-old male SD rats (Orient Bio) were used, and test groups are shown in Table 5 below.

[Table 5]

| Test group | | Test material information (per rat) | | | Number of animals |
|---|---|---|---|---|---|
| | | Administered material | mRNA (μg) | Administered amount | |
| G1 | Negative control | Phosphate-buffered saline (PBS) | 0 | 500 μL/rat | 5 each, 35 in total |
| G2 | Test group 1 | 1 HD EG-COVID | 200 | 500 μL/rat | 5 each, 35 in total |
| G3 | Test group 2 | 0.025 HD EG-COVID | 5 | 500 μL/rat | 5 each, 35 in total |

[Table 6] Glossary

| No. | Organs | Abbreviation |
|---|---|---|
| 1 | Left thigh muscle, Injection site left | ISL |
| 2 | Right thigh muscle, Non-injection site right | ISR |
| 3 | Kidney | K |
| 4 | Testis | TS |
| 5 | Brain | B |
| 6 | Heart | H |
| 7 | Spleen | S |
| 8 | Liver | L |
| 9 | Proximal popliteal lymph node | LNP |
| 10 | Distal axillary lymph node | LND |
| 11 | Lung | Lug |
| 12 | Eye | E |
| 13 | Serum | SR |

The serum and tissue samples were obtained from rats in the test groups, and total RNA was extracted using an RNeasy Micro kit (QIAGEN/74004) for serum and tissue according to the test method recommended by the manufacturer, and the concentration and yield of total RNA were determined using Nanodrop (Thermo Fisher).

[Table 7] Primers & probes for RT-qPCR (Thermo Fisher)

| Type | Sequence (5'->3') | bp | Product (bp) | Location |
|---|---|---|---|---|
| **Forward** | CCTGCGCGAGTTCGTGTT (SEQ ID NO: 9) | 18 | 61 | 612-672, S1 |
| **Reverse** | GGTGTGCTTGCTGTAGATCTTGA (SEQ ID NO: 10) | 23 | | |
| **Probe** | AAGAACATCGACGGCTAC (SEQ ID NO: 11) | 18 | | |

GAPDH primer and probe sets (Thermo Fisher) were used for rat GAPDH as a reference gene, and VIC dye was tagged at the 5' end of the primer.

[Table 8]

| GAPDH assay ID | GAPDH gene identification number | Product (bp) |
|---|---|---|
| **Rn01749022_g1** | NIM_017008.4 | 60 |

As shown in Tables 9 and 10 below, in a 96-well plate (Invitrogen/A28138) or a 384-well plate (Invitrogen/A28140), sample 1 (tissue-derived RNA), TaqPath 1-step multiplex master mix (hereinafter 'master mix', (Thermo Fisher/A28523)), primer & probe, GAPDH assay mix, and nuclease-free water each were dispensed using a multichannel pipette to reach a final volume of 15 μL, and qRT-PCR reaction was performed.

[Table 9]

| Component | Working Conc. | Sample |
|---|---|---|
| 4x Master mix | 1x | 3.75 |
| 60x CV-SF-614Gm primer & probe | 0.2x | 0.06 |
| 60x GAPDH assay mix | 0.2x | 0.5 |
| Total RNA | 25 ng | 5 |
| Nuclease-free water | - | 5.69 |

(continued)

| Component | Working Conc. | Sample |
|---|---|---|
| Total volume (μL) | | 15 |

[Table 10]

| qRT-PCR conditions | | | | |
|---|---|---|---|---|
| | Step | Cycles | Temperature (°C) | Time |
| 1 | Incubation | 1 | 25 | 2 min |
| 2 | Reverse transcription | 1 | 53 | 10 min |
| 3 | Polymerase activation | 1 | 95 | 2 min |
| 4 | Amplification | 40 | 95 | 15 sec |
| | | | 60 | 1 min |

The reaction results for each specimen were calculated as below to confirm the amount of CV-SF-614Gm to GAPDH.

CV-SF-614Gm to GAPDH Ct (ΔR) = Average Ct of CV-SF-614Gm - Average Ct of GAPDH

Also, for accurate comparison, ΔR was converted into an index.

$$Relative\ value\ of\ \Delta R\ index = \frac{1}{2^{\Delta R}}$$

Thereafter, the biodistribution pattern of CV-SF-614Gm was analyzed by drawing graphs by group, time, and tissue.

Based on results of measurement of the amount of CV-SF-614Gm to GAPDH in 12 major organs and serum including the site of administration, CV-SF-614Gm was detected only in the left thigh muscle (ISL) corresponding to the site of administration.

The relative index value of CV-SF-614Gm to GAPDH in ISL was observed to be a maximum of 57% after 2 hours of EG-COVID administration and a maximum of 70% after 6 hours. CV-SF-614Gm was detected to be the highest at 6 hours, gradually decreased after 6 hours, and was not detected after 72 hours. During the observation period, CV-SF-614Gm was not confirmed in major organs other than the site of administration (FIGs. 8a and 8b).

**Example 12: Evaluation of neutralizing antibody titer (plaque reduction neutralization test)**

In order to evaluate the neutralizing antibody titer of EG-COVID, the following test was performed to confirm the protective ability against SARS-CoV-2 (NCCP 43326, Wuhan-derived virus).

As test animals, 6-week-old female B6C3F1/slc mice (n=6/group) (Orient Bio) were used, and negative control and EG-COVID including CV-SF-614Gm as a test group were administered to the animals twice at 3-week intervals, and blood was collected 2 weeks later.

Virus experiments were performed by PRNT at Masan National Hospital, which has a BL3 facility.

A VERO 76 cell line (ATCC CRL-1587™) was seeded at $8\times10^5$ cells/well in a 6-well plate and then cultured for 24 hours to prepare for sub-confluence. Serum from the EG-COVID-administered group and serum from the negative control group were diluted 1/10 in serum-free DMEM, followed by 2-fold serial dilution up to 1/20,480, and 105 μL of the diluted serum was mixed at 1:1 with 400 pfu/$10^5$ μL of virus (SARS-CoV-2 (NCCP 43326)) solution and then cultured at 37°C for 1 hour.

The culture media of the VERO 76 cell line was removed, followed by washing with PBS, addition of 200 μL

of a mixed solution of the serum and virus, shaking at intervals of 15 minutes, and culture at 37°C for 90 minutes to allow virus infection. Thereafter, the culture media was removed, followed by washing with PBS to remove uninfected virus, after which the cells were covered with DMEM (Gibco) containing 2% FBS and 1% agarose, followed by culture in an incubator at 37°C for 3 days. After completion of culture, 4% formaldehyde solution was applied on agarose and fixed at room temperature for 1 hour, agarose was carefully removed, and the fixed cell layer was stained with 0.5% crystal violet (in 20% methanol) solution. After washing three times with PBS, the plate was completely dried. The infected cells stained with crystal violet (hereinafter referred to as 'plaque') were counted and compared with the non-neutralized control group, from which the virus infection inhibitory efficiency was determined, and the equation thereof is represented below.

$$Virus\ infection\ inhibitory\ efficiency\ (\%) = \frac{plaque\ number\ of\ control*\ plate - plaque\ number\ of\ sample\ plate}{plaque\ number\ of\ control*\ plate} \times 100$$

(Control is a group that does not neutralize the virus)

The neutralizing antibody titer was determined as $IC_{50}$ titer ($log_{10}$) by calculating the dilution factor at which the reduction rate was 50% using nonlinear regression of GraphPad Prism software based on the virus infection inhibitory efficiency.

Thereby, as shown in FIG. 9A, for infection with SARS-CoV-2 wild-type virus (NCCP43326, The National Culture Collection for Pathogens in Korea), in EG-COVID-administered group 1 (2.5 ug/mouse), EG-COVID-administered group 2 (5 ug/mouse), and EG-COVID-administered group 3 (10 μg/mouse), respective $IC_{50}$ titers ($log_{10}$) were 3.569±0.418, 4.039±0.357, and 4.375±0.443 (mean±standard deviation), indicating that the neutralizing antibody titer increased with an increase in the amount of mRNA.

In addition, as shown in FIG. 9B, it was confirmed that superior cross-immunity was also exhibited against Alpha and Beta variants as SARS-CoV-2 variants.

## Industrial Applicability

According to the present invention, a vaccine for preventing SARS-CoV-2 is capable of exhibiting superior vaccine effect due to superior stability and high immunogenicity *in vivo* without additional immune enhancers, and also of maintaining the vaccine effect even when prepared in a lyophilized formulation, and thus the vaccine is easy to store and use, thereby expecting excellent effects in the prevention of COVID-19.

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

## Sequence List Free Text

An electronic file is attached.

## Claims

1. A vaccine composition for preventing SARS-CoV-2 comprising mRNA encoding an S antigen of SARS-CoV-2 virus.

2. The vaccine composition according to claim 1, wherein the mRNA encoding the S antigen has a nucleotide sequence of SEQ ID NO: 1 or SEQ ID NO: 2.

3. The vaccine composition according to claim 1 or 2, further comprising liposomes or lipid nanoparticles.

4. The vaccine composition according to claim 3, wherein the liposomes or lipid nanoparticles comprise a cationic lipid.

5. The vaccine composition according to claim 4, wherein the liposomes or lipid nanoparticles further comprise a neutral lipid.

**6.** The vaccine composition according to claim 4 or 5, wherein the liposomes or lipid nanoparticles further comprise cholesterol.

**7.** The vaccine composition according to any one of claims 4 to 6, wherein the cationic lipid is at least one selected from the group consisting of dimethyldioctadecylammonium bromide (DDA), C12-200, 1,2-dioleoyl-3-trimethylammonium propane (DOTAP), 3β-[N-(N',N'-dimethylaminoethane)carbamoyl cholesterol (DC-Chol), 1,2-dioleoyloxy-3-dimethylammonium propane (DODAP), 1,2-di-O-octadecenyl-3-trimethylammonium propane (DOTMA), 1,2-dimyristoleoyl-sn-glycero-3-ethylphosphocholine (14:1 Ethyl PC), 1-palmitoyl-2-oleoyl-sn-glycero-3-ethylphosphocholine (16:0-18:1 Ethyl PC), 1,2-dioleoyl-sn-glycero-3-ethylphosphocholine (18:1 Ethyl PC), 1,2-distearoyl-sn-glycero-3-ethylphosphocholine (18:0 Ethyl PC), 1,2-dipalmitoyl-sn-glycero-3-ethylphosphocholine (16:0 Ethyl PC), 1,2-dimyristoyl-sn-glycero-3-ethylphosphocholine (14:0 Ethyl PC), 1,2-dilauroyl-sn-glycero-3-ethylphosphocholine (12:0 Ethyl PC), N1-[2-((1S)-1-[(3-aminopropyl)amino]-4-[di(3-aminopropyl)amino]butylcarboxamido)ethyl]-3,4-di[oleyloxy]-benzamide (MVL5), 1,2-dimyristoyl-3-dimethylammonium-propane (14:0 DAP), 1,2-dipalmitoyl-3-dimethylammonium-propane (16:0 DAP), 1,2-distearoyl-3-dimethylammonium-propane (18:0 DAP), N-(4-carboxybenzyl)-N,N-dimethyl-2,3-bis(oleoyloxy)propan-1-aminium (DOBAQ), 1,2-stearoyl-3-trimethylammonium-propane (18:0 TAP), 1,2-dipalmitoyl-3-trimethylammonium-propane (16:0 TAP), 1,2-dimyristoyl-3-trimethylammonium-propane (14:0 TAP), and N4-cholesteryl-spermine (GL67).

**8.** The vaccine composition according to claim 5, wherein the neutral lipid is at least one selected from the group consisting of 1,2-dimyristoyl-sn-glycero-3-phosphatidylcholine (DMPC), 1,2-dioleoyl-sn-glycero-3-phosphocholine (DOPC), 1,2-dioleoyl-sn-glycero-3-phosphoethanolamine (DOPE), 1,2-dipalmitoyl-sn-glycero-3-phosphocholine (DPPC), 1,2-distearoyl-sn-glycero-3-phosphocholine (DSPC), 1,2-dilinoleoyl-sn-glycero-3-phosphocholine (DLPC), phosphatidylserine (PS), phosphoethanolamine (PE), phosphatidylglycerol (PG), phosphoric acid (PA), phosphatidylcholine (PC), DOPI (1,2-dioleoyl-sn-glycero-3-phospho-(1'-myo-inositol)), and DSPI (1,2-distearoyl-sn-glycero-3-phosphoinositol).

**9.** The vaccine composition according to any one of claims 5 to 8, wherein a weight ratio of the cationic lipid to the neutral lipid is 1:9 to 9.5:0.5.

**10.** The vaccine composition according to claim 6, wherein a weight ratio of the cationic lipid to the cholesterol is 6:1 to 1:3.

**11.** The vaccine composition according to claim 10, wherein a weight ratio of the cationic lipid to the neutral lipid to the cholesterol is 1-9.5:0.5-9:0.05-3.

**12.** The vaccine composition according to claim 3, wherein the liposomes or lipid nanoparticles and mRNA have an N:P ratio of 0.2:1 to 1.4:1.

**13.** The vaccine composition according to any one of claims 1 to 12, further comprising an immune enhancer.

**14.** The vaccine composition according to claim 13, wherein the immune enhancer comprises at least one selected from the group consisting of PAMP, saponin, CpG DNA, lipoprotein, flagella, poly I:C, squalene, tricaprin, 3D-MPL, and detoxified lipooligosaccharide (dLOS).

**15.** The vaccine composition according to any one of claims 1 to 14, which is in a form of a lyophilized formulation.

**16.** A method of preparing a vaccine composition for preventing SARS-CoV-2, comprising adding a solution or buffer comprising liposomes or lipid nanoparticles to mRNA encoding an S antigen of SARS-CoV-2 virus or a solution or buffer comprising the same.

FIG. 1

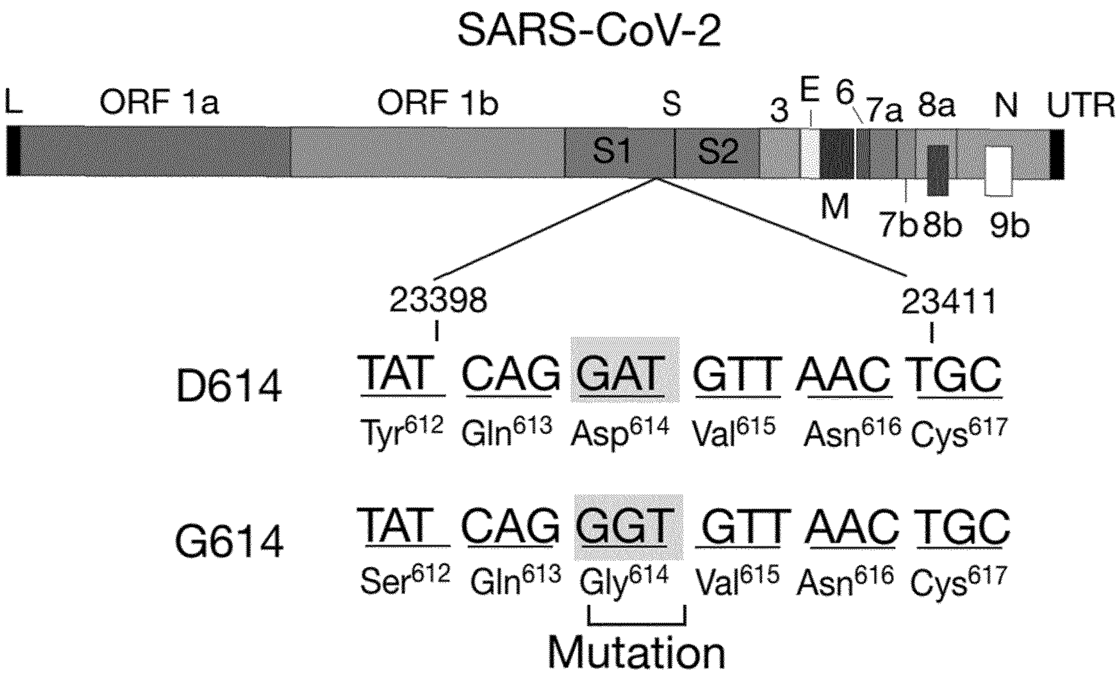
SARS-CoV-2
L
ORF 1a
ORF 1b
S
3
E
6
7a
8a
N
UTR
S1
S2
M
7b
8b
9b
23398
23411
D614
TAT CAG GAT GTT AAC TGC
Tyr612 Gln613 Asp614 Val615 Asn616 Cys617
G614
TAT CAG GGT GTT AAC TGC
Ser612 Gln613 Gly614 Val615 Asn616 Cys617
Mutation

FIG. 2

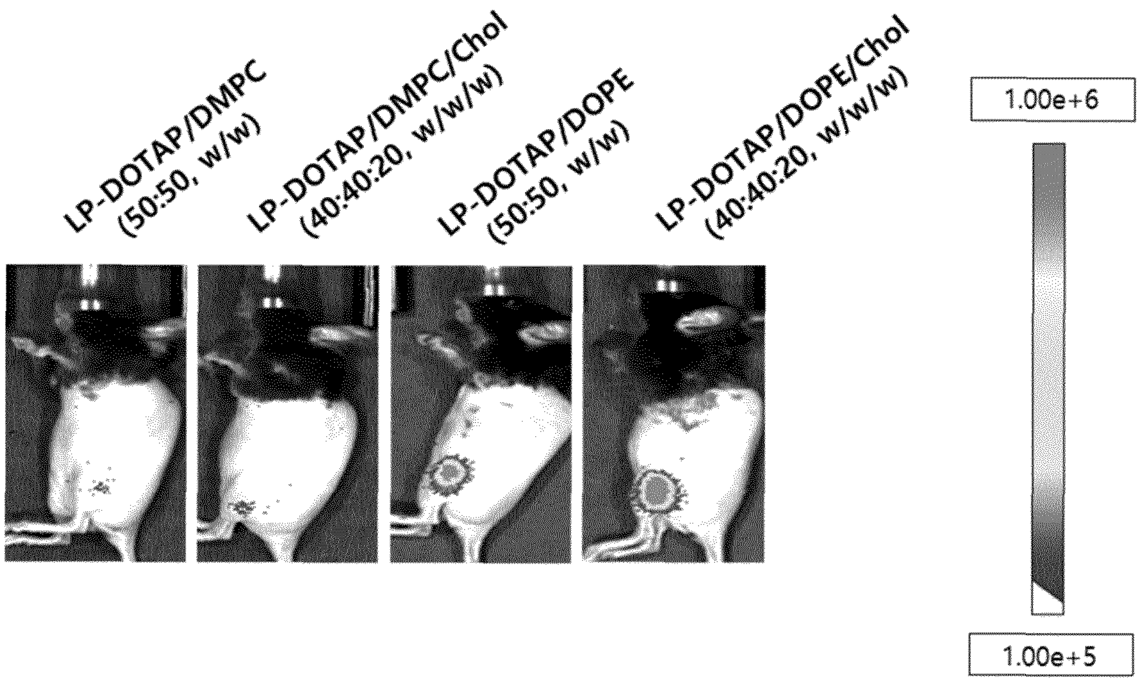
LP-DOTAP/DMPC (50:50, w/w)
LP-DOTAP/DMPC/Chol (40:40:20, w/w/w)
LP-DOTAP/DOPE (50:50, w/w)
LP-DOTAP/DOPE/Chol (40:40:20, w/w/w)
1.00e+6
1.00e+5

FIG. 3

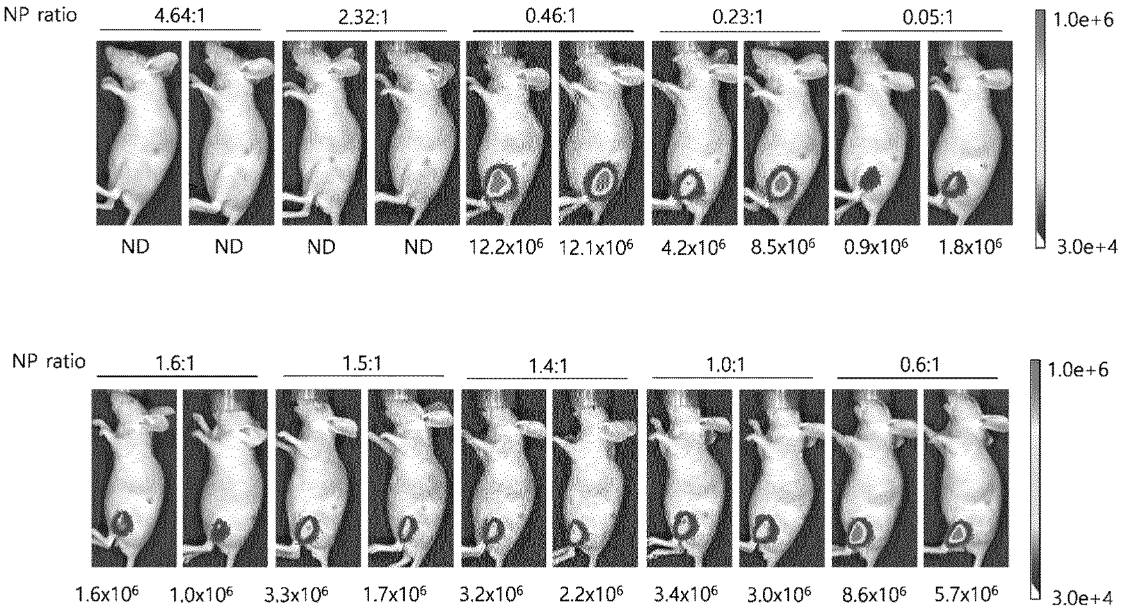
NP ratio
4.64:1
2.32:1
0.46:1
0.23:1
0.05:1
ND ND ND ND 12.2x10^6 12.1x10^6 4.2x10^6 8.5x10^6 0.9x10^6 1.8x10^6
1.0e+6
3.0e+4
NP ratio
1.6:1
1.5:1
1.4:1
1.0:1
0.6:1
1.6x10^6 1.0x10^6 3.3x10^6 1.7x10^6 3.2x10^6 2.2x10^6 3.4x10^6 3.0x10^6 8.6x10^6 5.7x10^6
1.0e+6
3.0e+4

FIG. 4

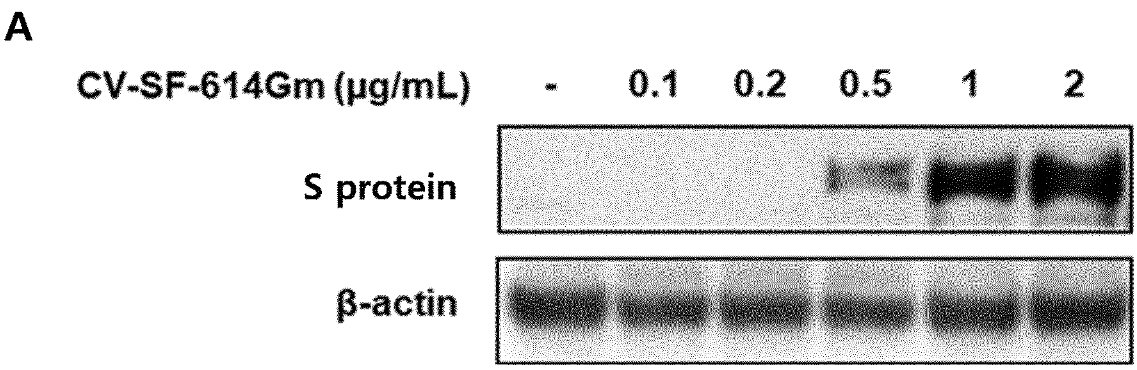
A
CV-SF-614Gm (μg/mL)
- 0.1 0.2 0.5 1 2
S protein
β-actin

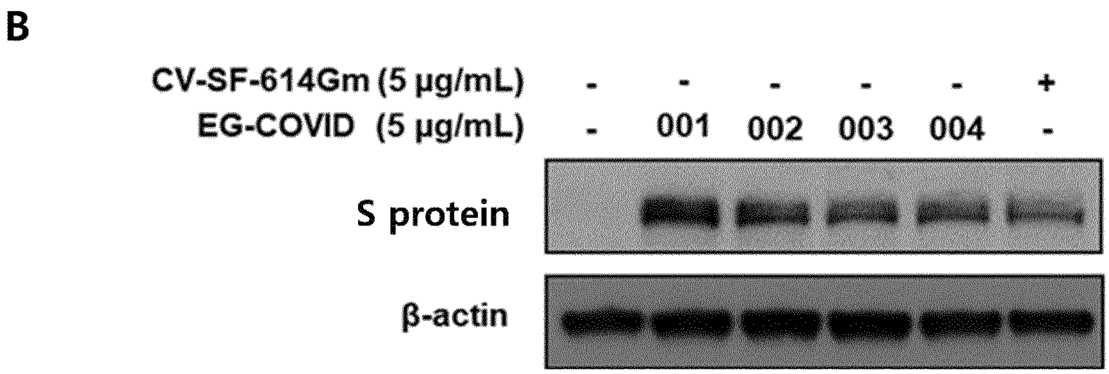
B
CV-SF-614Gm (5 μg/mL) - - - - - +
EG-COVID (5 μg/mL) - 001 002 003 004 -
S protein
β-actin

FIG. 5

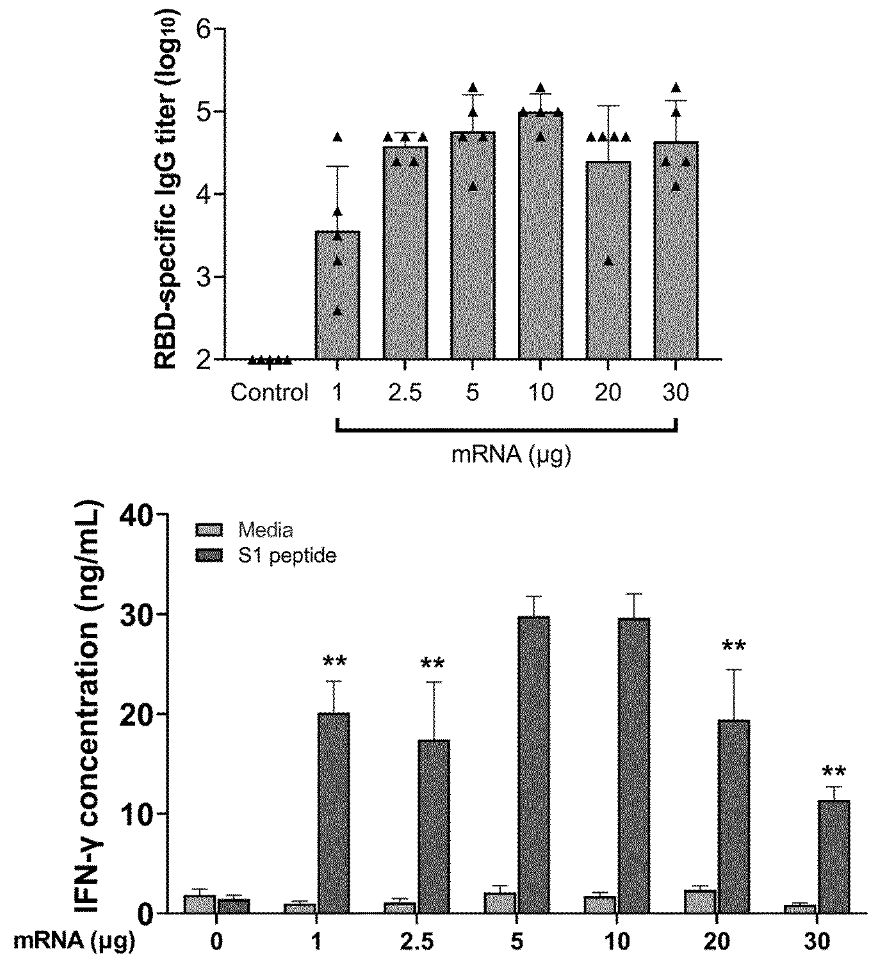


**p<0.01 compared with G4 (mRNA 5μg/mouse administered) by one-way ANOVA

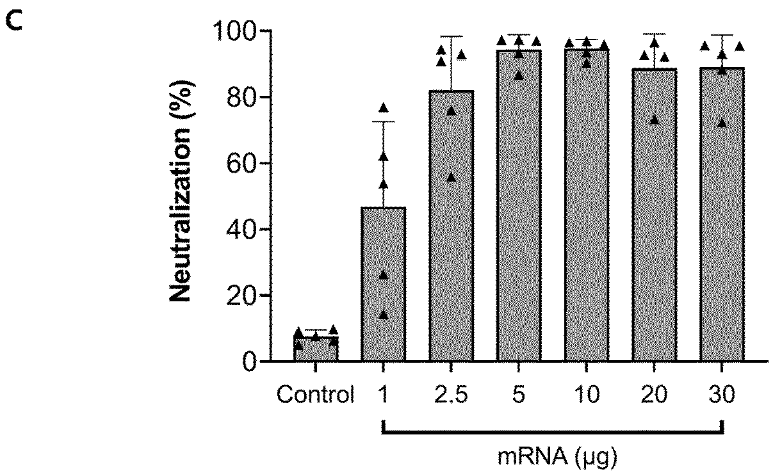

FIG. 6

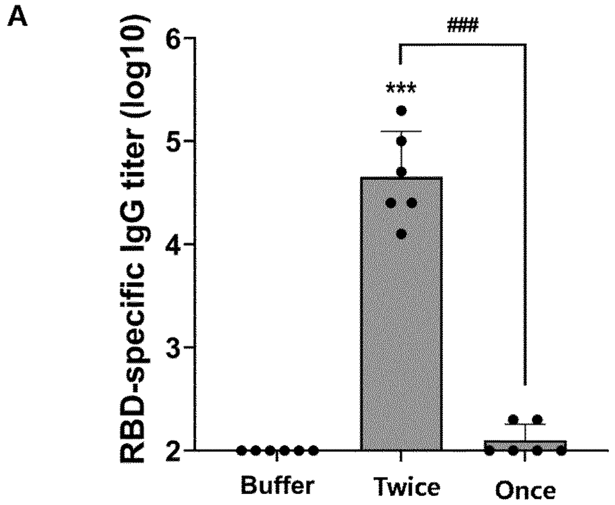


***p<0.001, compared with buffer; ###p<0.001 by Student's t-test

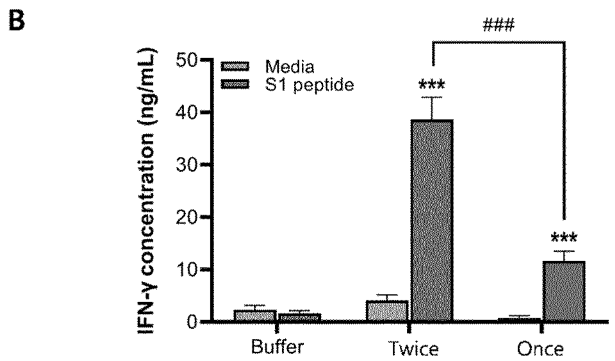


***p<0.001, compared with buffer; ###p<0.001 by Student's t-test

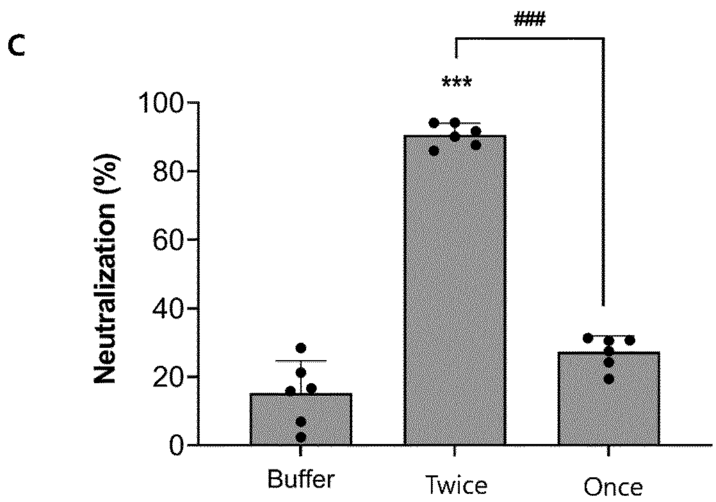


***p<0.001, compared with buffer; ###p<0.001 by Student's t-test

FIG. 7

A

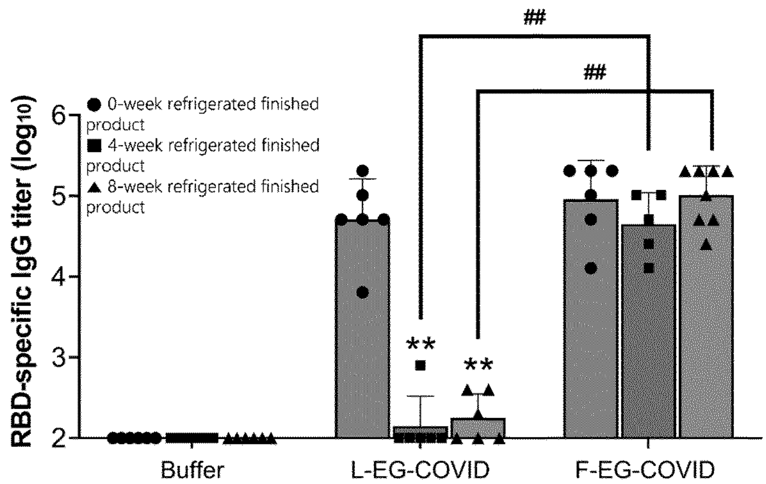


**p<0.01, compared with 0-week refrigerated finished product depending on type of formulation;
##p<0.01, compared with lyophilized finished product depending on storage period by one-way ANOVA

B

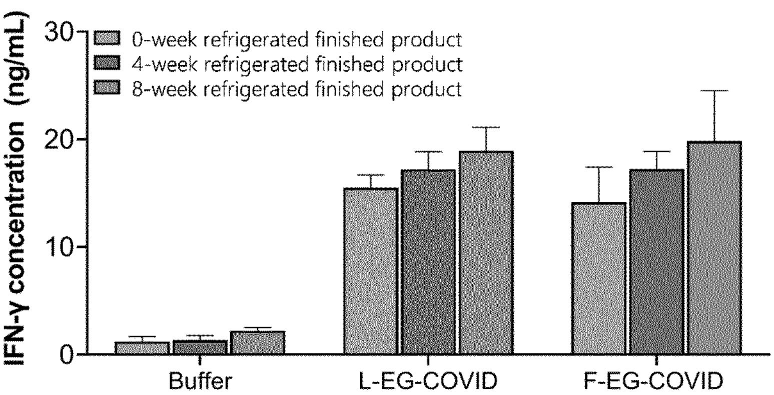


C

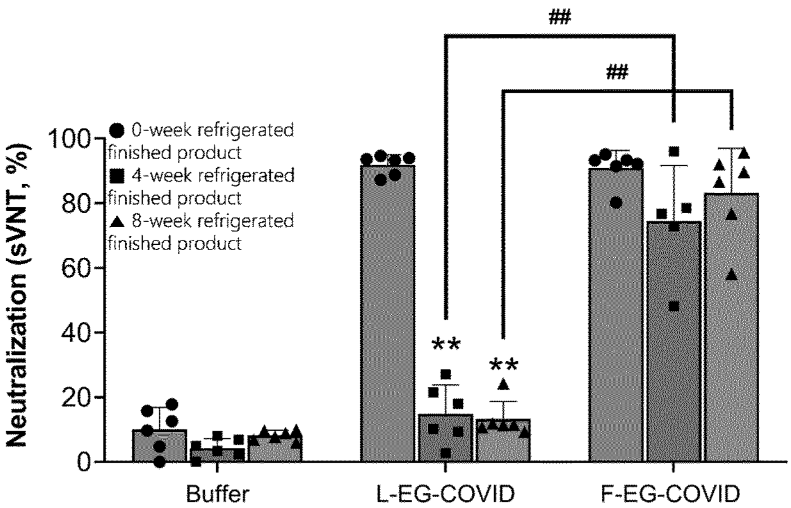


**p<0.01, compared with 0-week refrigerated finished product depending on type of formulation;
##p<0.01, compared with lyophilized finished product depending on storage period by one-way ANOVA

FIG. 8a

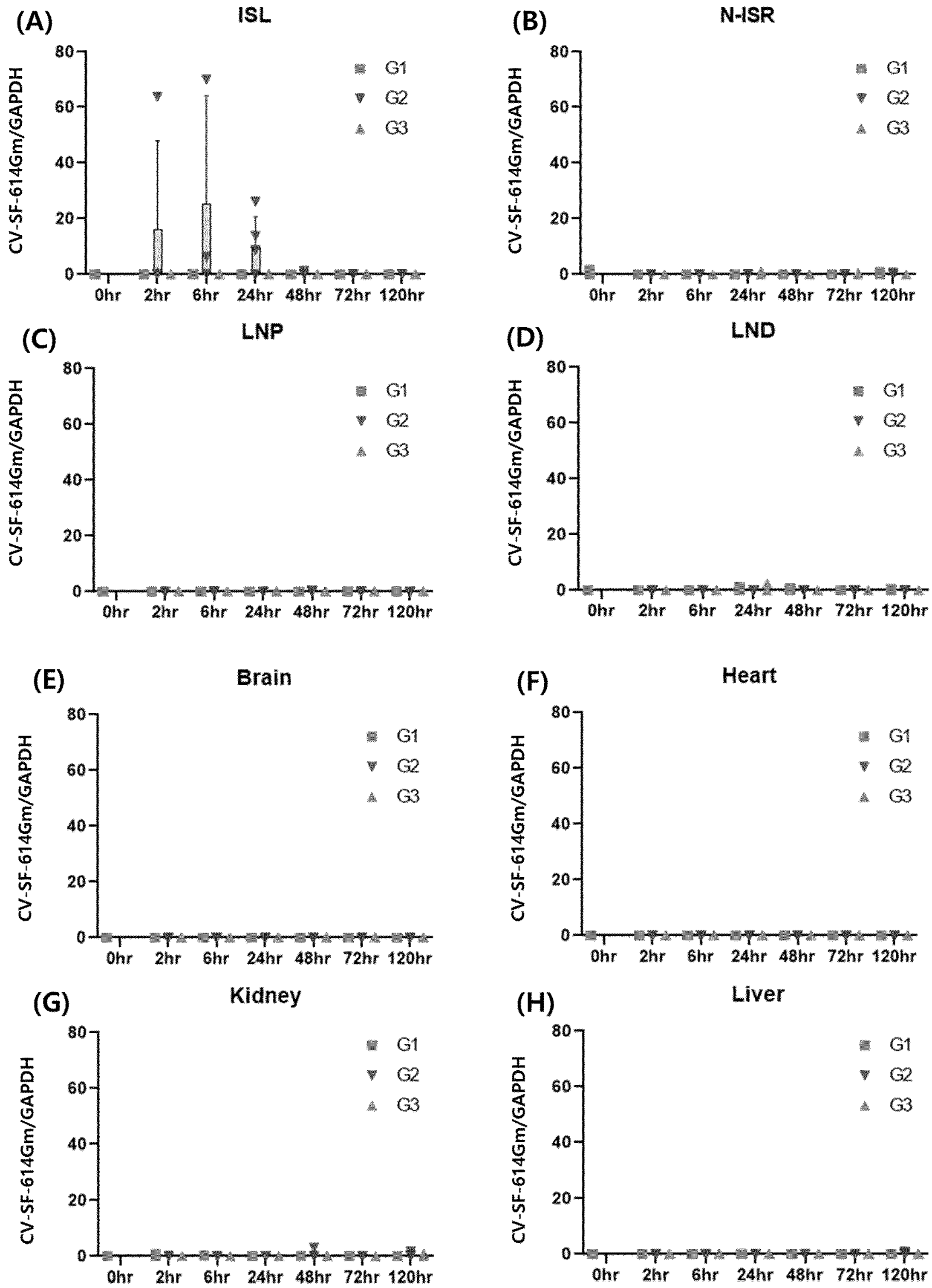
(A)
ISL
CV-SF-614Gm/GAPDH
80
60
40
20
0
0hr 2hr 6hr 24hr 48hr 72hr 120hr
G1
G2
G3
(B)
N-ISR
(C)
LNP
(D)
LND
(E)
Brain
(F)
Heart
(G)
Kidney
(H)
Liver

FIG. 8b

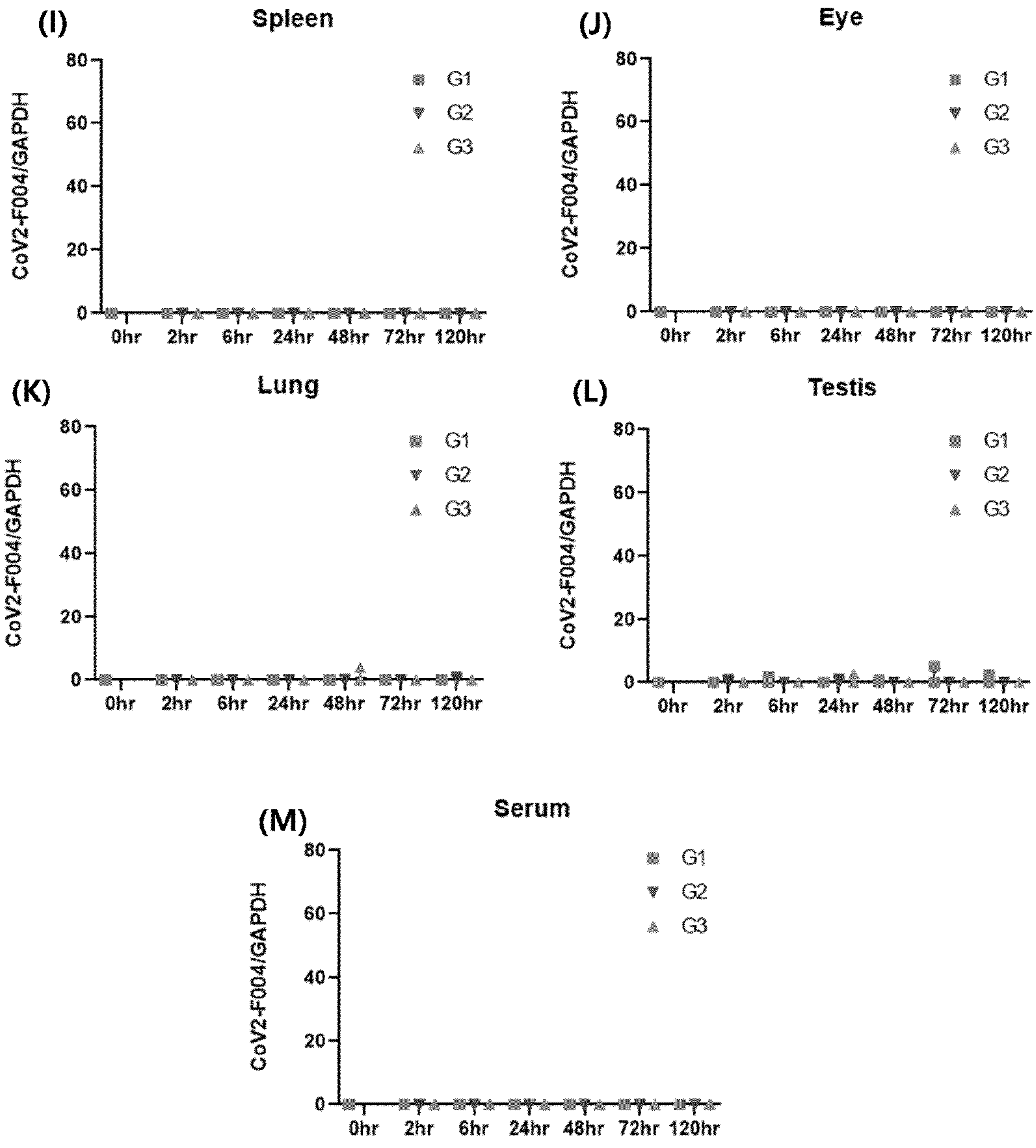
(I)
Spleen
(J)
Eye
(K)
Lung
(L)
Testis
(M)
Serum
CoV2-F004/GAPDH
G1
G2
G3
0
20
40
60
80
0hr
2hr
6hr
24hr
48hr
72hr
120hr

FIG. 9

A

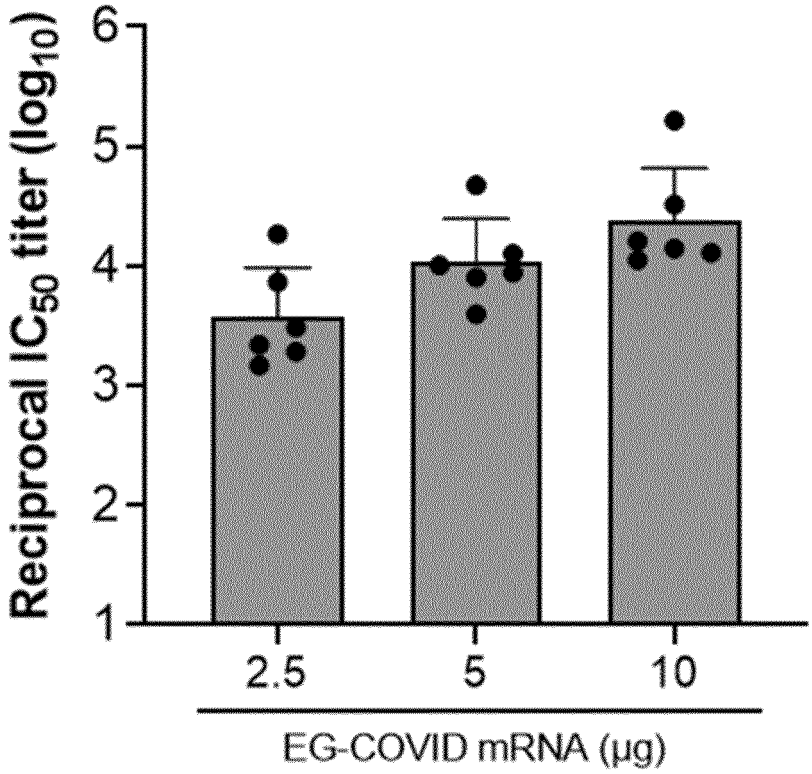
Reciprocal IC50 titer (log10)
6
5
4
3
2
1
2.5
5
10
EG-COVID mRNA (μg)

B

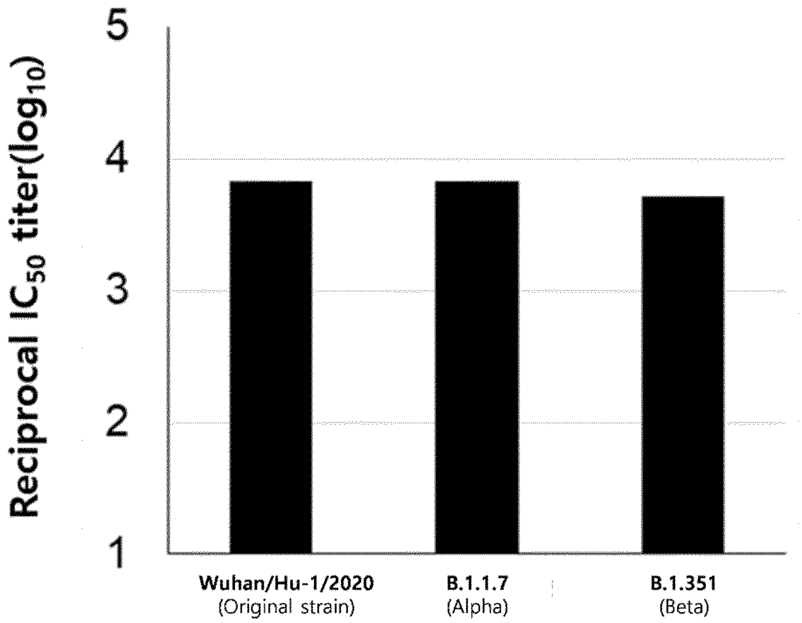
Reciprocal IC50 titer(log10)
5
4
3
2
1
Wuhan/Hu-1/2020
(Original strain)
B.1.1.7
(Alpha)
B.1.351
(Beta)

**INTERNATIONAL SEARCH REPORT**

International application No.
**PCT/KR2021/016460**

**A. CLASSIFICATION OF SUBJECT MATTER**

**A61K 39/215**(2006.01)i; **A61K 9/127**(2006.01)i; **A61K 9/51**(2006.01)i; **A61K 47/28**(2006.01)i; **A61K 9/19**(2006.01)i; **A61P 31/14**(2006.01)i; **A61K 48/00**(2006.01)i; **A61K 39/00**(2006.01)i

According to International Patent Classification (IPC) or to both national classification and IPC

**B. FIELDS SEARCHED**

Minimum documentation searched (classification system followed by classification symbols)

A61K 39/215(2006.01); A61K 39/00(2006.01); A61K 39/12(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: SARS-CoV-2, 리포좀(liposome), 지질나노입자(lipid nanoparticle), 백신(vaccine)

**C. DOCUMENTS CONSIDERED TO BE RELEVANT**

| Category* | Citation of document, with indication, where appropriate, of the relevant passages | Relevant to claim No. |
|---|---|---|
| X | LIU, L. et al. A translatable subunit nanovaccine for COVID-19. ChemRxiv. 2020, pp. 1-15.<br>See abstract; and pages 2 and 10-11. | 1,3-6,8,10-12,16 |
| X | HUANG, W.-C. et al. SARS-CoV-2 RBD Neutralizing Antibody Induction is Enhanced by Particulate Vaccination. Adv Mater. 2020, vol. 32, thesis no.:2005637, pp. 1-11.<br>See abstract; and page 8. | 1 |
| A | KR 10-2086987 B1 (EYEGENE INC.) 10 March 2020 (2020-03-10)<br>See entire document. | 1,3-6,8,10-12,16 |
| A | SCHWENDENER, R. A. Liposomes as vaccine delivery systems: a review of the recent advances. Ther Adv Vaccines. 2014, vol. 2, no. 6, pp. 159-182.<br>See entire document. | 1,3-6,8,10-12,16 |
| PX | HONG, H. C. et al. An mRNA vaccine against SARS-CoV-2: Lyophilized, liposome-based vaccine candidate EG-COVID induces high levels of virus neutralizing antibodies. bioRxiv. 22 March 2021.<br>See abstract; and lines 217-259. | 1,3-6,8,10-12,16 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 April 2022** | **20 April 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2021/016460**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **2,14**
because they relate to parts of the international application that do not comply with the prescribed requirements to such an extent that no meaningful international search can be carried out, specifically:

- With respect to claim 2 pertaining to SEQ ID NO: 1 or 2, the description discloses only a sequence of an image form, and thus the filing sequence listing in the form of an Annex C/ST. 25 text file for international search is requested, but since the sequence listing has not been filed, meaningful international search therefor cannot be carried out.
- Claim 14 refers to claim 13 which violates the manner of referring to a dependent claim (PCT Rule 6.4(a)), and thus is unclear.

3. ☑ Claims Nos.: **7,9,13,15**
because they are dependent claims and are not drafted in accordance with the second and third sentences of Rule 6.4(a).

Form PCT/ISA/210 (continuation of first sheet) (July 2019)

**INTERNATIONAL SEARCH REPORT**
**Information on patent family members**

International application No.
**PCT/KR2021/016460**

| Patent document cited in search report | Publication date (day/month/year) | Patent family member(s) | Publication date (day/month/year) |
|---|---|---|---|
| KR 10-2086987 B1 | 10 March 2020 | AU 2017-351899 A1 | 06 June 2019 |
| | | AU 2017-351899 B2 | 03 December 2020 |
| | | AU 2017-351900 A1 | 06 June 2019 |
| | | AU 2017-351900 B2 | 26 November 2020 |
| | | CN 110461355 A | 15 November 2019 |
| | | CN 110545840 A | 06 December 2019 |
| | | EA 201991076 A1 | 31 October 2019 |
| | | EA 201991081 A1 | 31 October 2019 |
| | | EP 3533463 A1 | 04 September 2019 |
| | | EP 3533464 A1 | 04 September 2019 |
| | | JP 2020-500213 A | 09 January 2020 |
| | | JP 2020-502258 A | 23 January 2020 |
| | | JP 6963018 B2 | 05 November 2021 |
| | | JP 6963019 B2 | 05 November 2021 |
| | | KR 10-2018-0047054 A | 10 May 2018 |
| | | KR 10-2019-0000586 A | 03 January 2019 |
| | | KR 10-2019-0021938 A | 06 March 2019 |
| | | KR 10-2019-0021939 A | 06 March 2019 |
| | | KR 10-2042993 B1 | 11 November 2019 |
| | | KR 10-2086986 B1 | 10 March 2020 |
| | | KR 10-2086988 B1 | 10 March 2020 |
| | | US 10874733 B2 | 29 December 2020 |
| | | US 10918709 B2 | 16 February 2021 |
| | | US 2019-0255171 A1 | 22 August 2019 |
| | | US 2019-0290749 A1 | 26 September 2019 |
| | | WO 2018-080252 A1 | 03 May 2018 |
| | | WO 2018-080253 A1 | 03 May 2018 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1509456
- KR 2042993
- KR 20140097215


### Non-patent literature cited in the description


- **MATTHEW Z T et al.** *Nature Reviews Immunology,* 2020, vol. 20, 363-374
- **JESPER PALLESEN et al.** *PNAS,* 2017, https://doi.org/10.1073/PNAS.1707304114
- **PLANTE, J. A et al.** *Nature,* 2020, https://doi.org/10.1038/s41586-020-2895-3
- **WOLFF JA et al.** *Science,* 1990, vol. 247, 1465-8
- **SAYOUR EJ et al.** *J Immunother Cancer,* 2015, vol. 3, 13
- **FILION M. C ; PHILLIPS N. C.** *Biochimica et Biophysica Acta (BBA)-Biomembranes,* 1997, vol. 1329 (2), 345-356
- *Therapeutic Advances in Vaccines,* vol. 2 (6), 159-82
- **DOWHAN W et al.** *New Comprehensive Biochemistry,* 2002, vol. 36 (4), 1-35
- **LEUNG A-KK et al.** *The Journal of Physical Chemistry B,* 2015, vol. 119 (28), 8698-8706
- **KAUFFMAN KJ et al.** *Nano letters,* 2015, vol. 15 (11), 7300-7306
- Antisense drug technology; Principles, Strategy, and Application. CRC press, 2007, vol. 253
- *Appl. Chem. Eng.,* 2017, vol. 28 (2), 177-185